# FASCICULE DE BREVET EUROPEEN

(11) **EP 0 486 661 B1**
(45) Date de publication et mention de la délivrance du brevet: **07.02.1996**
(21) Numéro de dépôt: 91911286.2
(22) Date de dépôt: 11.06.1991
(51) Int. Cl.: C12Q 1/68

(54) **PROCEDE DE DETECTION D'UNE SEQUENCE NUCLEOTIDIQUE SELON LA TECHNIQUE D'HYBRIDATION SANDWICH**
VERFAHREN ZUM NUKLEOTIDSEQUENZENNACHWEIS MITTELS TECHNIK DER SANDWICH-HYBRIDISIERUNG
METHOD FOR DETECTING A NUCLEOTIDE SEQUENCE BY SANDWICH HYBRIDIZATION

(30) Priorité: 11.06.1990 FR 9007249
(43) Date de publication de la demande: 27.05.1992
(73) Titulaire: BIO MERIEUX, Société anonyme, F-69280 Marcy l'Etoile (FR)
(72) Inventeur: CROS, Philippe, F-69005 Lyon (FR); ALLIBERT, Patrice, F-69290 Grézieu-la-Varenne (FR); MALLET, François, F-69100 Villeurbanne (FR); MABILAT, Claude, F-69100 Villeurbanne (FR); MANDRAND, Bernard, F-69100 Villeurbanne (FR)
(74) Mandataire: Tonnellier, Jean-Claude
(86) Numéro de dépôt international: FR9100468
(87) Numéro de publication internationale: WO9119812

(56) Documents cités:
- EP-A- 0 202 758
- EP-A- 0 269 764
- EP-A- 0 405 592
- EP-A- 0 420 260
- WO-A-86/07387
- WO-A-88/01302
- WO-A-88/04301
- WO-A-90/01069
- Clinical Chemistry, vol. 31, no. 9, 1985, R. Polsky-Cynkin et al.: "Use of DNA immobilized on plastic and agarose supports to detect DNA by sandwich hybridization", pages 1438-1443, voir l'article en entier; particulièrement abrégé; page 1439, colonne 1, ligne 55 - colonne 2, ligne 14; figure 2 (cité dans la demande)
- Clinical Chemistry, vol. 32, no. 9, 1986, P. Helsingius et al.: "Solid-phase immunoassay of digoxin by measuring time-resolved fluorescence", pages 1767- 1769, voir page 1767, colonne 1, ligne 40 - page 1768, colonne 1, ligne 21

## Description

La présente invention a pour objet un procédé de détection d'une séquence nucléotidique simple brin dans un échantillon la contenant ou susceptible de la contenir, selon la technique d'hybridation sandwich. Ce procédé est applicable notamment au diagnostic des maladies infectieuses ou génétiques, ainsi qu'au typage cellulaire.

On sain que l'une des propriétés caractéristiques des acides nucléiques est la possibilité d'interagir avec une séquence complémentaire par l'intermédiaire de liaisons hydrogène et de former ainsi un hybride stable selon les lois d'appariement A-T et G-C.

Un acide nucléique peut donc être utilisé comme sonde pour mettre en évidence, dans un échantillon, une séquence nucléique (appelée "cible") contenant une séquence complémentaire de celle de la sonde. Le marquage de l'hybride formé entre la cible et la sonde permet la détection et la quantification de la cible dans l'échantillon. Cette technique a été utilisée par SOUTHERN E.M. pour l'analyse de fragments d'ADN après séparation par gel d'électrophorèse : J. Mol. Biol 98,503 (1975).

Une modification de cette technique, qui présente notamment l'avantage de ne pas nécessiter la purification de l'échantillon contenant la cible, consiste à utiliser un protocole dit "sandwich". Une première sonde nucléotidique (sonde de capture) fixée sur un support solide sert à capter le gène ou le fragment de gène à détecter dans l'échantillon. Une deuxième sonde (sonde de détection), complémentaire d'une autre région de la cible permet la détection par l'intermédiaire d'un marqueur tel qu'un marqueur radioactif ; voir par exemple DUNN A.R. et HASSEL J.A., Cell, 12, 23, (1977) ; RANKI M. et al., Gene, 21,77 (1983) ; PALVA A. et al., FEMS Microbiol. Lent. 23,83 (1984) POLSKY-CYNKIN R. et al., Clin. Chem. 31, 1438 (1985).

La méthode d'hybridation sandwich peut être réalisée en ajoutant en une seule étape l'échantillon à analyser et la sonde de détection dans un conteneur où se trouve la sonde de capture fixée sur le support. Il s'agit dans ce cas de la méthode d'hybridation sandwich dite "simultanée" ; voir par exemple les articles de RANKI et al., PALVA et al., POLSKY-CYNKIN et al., déjà cités.

La technique d'hybridation sandwich peut également être effectuée en deux étapes ; voir par exemple LANGDALE J.A. et al., Gene 36 201 (1985) et ZOLG J.W. et al., Mol. Biochem. Parasitol, 22,145 (1987).

Pour permettre l'utilisation de ces techniques d'hybridation dans des essais de routine, il est nécessaire de s'affranchir du marquage radioactif de la sonde de détection. on a proposé dans ce but divers systèmes faisant appel par exemple à un haptène reconnu par un anticorps, ou par une protéine affine. On a proposé en particulier diverses méthodes de marquage d'ADN ou d'ARN par la biotine ou ses dérivés ; voir notamment les demandes de brevet européen 0285057, 0285058, 0286898 et 0097373 ; FORSTER et al., Nucleic Acid Res. 13,745 (1985) ; CHOLLET A. et KAWASHIMA E.H., Nucleic Acid Res. 13,1529 (1985) ; CHIN B.C. et al., Nucleic Acid Res. 11,6513 (1983) ; COCUZZA A.J., Tetrahedron Lett. 30,6287 (1989). Toutefois, les systèmes de détection utilisant des méthodes de marquage non radioactif manquent encore de sensibilité ; voir par exemple ZOLG et al., article cité.

Par ailleurs, le diagnostic des maladies génétiques, le typage cellulaire et même dans certains cas l'identification de virus mutants nécessitent la détection de mutations ponctuelles dans le génome. Dans de tels cas, la mise au point de sondes de capture suffisamment sensibles pour détecter une mutation ponctuelle dans la région de la cible complémentaire de la séquence de la sonde de capture, est difficile. Les systèmes de sondes commercialisés actuellement pour réaliser ce type de détection sont peu pratiques car chaque système commercial doit être mis en oeuvre à une température donnée qui varie avec le système utilisé, ce qui empêche de faire des déterminations variées en opérant sur des appareils automatiques travaillant à une température unique.

La présente invention a pour objet un procédé de détection de séquences nucléiques, selon la technique d'hybridation sandwich, ayant une sensibilité suffisante pour permettre l'utilisation d'une sonde de détection non radioactive. Cela est rendu possible grâce à l'utilisation de sondes de capture de très courte taille dont on a découvert qu'elles confèrent au test sandwich une grande spécificité tout en conservant une bonne sensibilité et qu'elles permettent de détecter et de différentier des séquences homologues à un nucléotide près. L'utilisation d'oligo-nucléotides de courte taille facilite la construction des sondes en grande quantité avec un rendement acceptable, et permet de disposer d'un grand éventail de sélectivités. Cela est particulièrement utile dans le cas du typage HLA où les mutations définissant le type cellulaire sont localisées sur de courtes portions du gène.

On a en outre découvert qu'il est possible de réaliser l'adsorption passive directe, sur un support polymère tel que le polystyrène, de sondes oligo-nucléotidiques de très courte taille (jusqu'à un minimum de 11 nucléotides), ainsi que la fixation passive de sondes encore plus courtes (jusqu'à un minimum de 9 nucléotides) par l'intermédiaire d'une protéine à laquelle la sonde est liée de façon covalente. Il s'agit d'un résultat surprenant puisque jusqu'à présent, on utilisait généralement, comme sondes de capture, des séquences comportant plus de 100 bases (généralement plusieurs centaines de bases) ou des séquences de 15 à 100 nucléotides liées de façon covalente au support solide (demande de brevet WO 88/01302), et aussi puisque les séquences oligo-nucléotidiques (dT)₁₅ ne se fixent pas de façon passive (c'est-à-dire par adsorption) sur le polystyrène ; voir LACY M.J. et VOSS E.W., J. of Immunol. Methods, 116, 87-98 (1989). D'autres auteurs cherchaient à favoriser la fixation de la sonde de capture en saturant le support de polystyrène avec du poly(dT)₄₀₀₀ et en couplant la sonde de capture, comportant plusieurs dizaines de bases, à une "queue" de poly(dA) ayant de 30 à plus de 100 motifs ; voir MORRISSEY D.V. et COLLINS M.L., Molecular and Cellular Probes, 3, 1 89-207 (1989). Selon l'expérience de la demanderesse, la saturation du support solide par du poly(dT) n'améliore pas la détection.

En outre, grâce à l'utilisation de sondes de courte taille procurant une sensibilité accrue, le procédé de l'invention permet de choisir à volonté une température de travail prédéterminée, et àe construire dans chaque cas des sondes de longueur adaptée pour pouvoir effectuer les déterminations des cibles les plus diverses à une même température de travail prédéterminée. On conçoit aisément la simplification de procédure et les possibilités d'automatisation qui en résultent.

La présente invention a donc pour objet un procédé de détection d'une séquence nucléotidique simple brin dans un échantillon la contenant ou susceptible de la contenir, selon la technique d'hybridation sandwich avec une sonde de capture fixée de façon passive sur un support solide et une sonde de détection marquée avec un marqueur non radioactif, les sondes de capture et de détection étant capables d'hybridation, respectivement, avec deux régions non chevauchantes de la séquence nucléotidique-cible recherchée, caractérisé par le fait que la sonde de capture, contenant de 9 à 30 nucléotides, notamment de 9 à 25 nucléotides, et en particulier de 9 à 20 nucléotides, est fixée de façon passive sur ledit support solide qui est réalisé en matériau hydrophobe.

Lorsque la sonde de capture contient au moins il nucléotides, il est généralement possible de la fixer de façon non spécifique (c'est-à-dire par adsorption) sur le support, généralement réalisé en polymère hydrophobe tel que le polystyrène ou un copolymère à base de styrène, notamment un copolymère butadiène-styrène ou analogue. Parmi les copolymères de styrène, on citera ceux qui contiennent au moins 10% en poids de motifs styrène, et en particulier ceux qui contiennent au moins 30%, en poids, de motifs styrène. Le matériau constituant le support solide utilisé selon l'invention peut en outre être constitué d'un mélange de polystyrène et/ou de copolymère de styrène (en particulier butadiène-styrène) avec un autre polymère permettant notamment d'améliorer les propriétés du support solide (propriétés mécaniques, transparence, etc ...). Ledit autre polymère est par exemple un polymère styrène-acrylonitrile, styrène-méthacrylate de méthyle, un polypropylène, un polycarbonate ou analogue. Le mélange de polymères constituant le support solide contient alors au moins 10 % en poids, et de préférence au moins 30 % en poids, de motifs styrène. On peut utiliser comme support solide un support conique commercialisé par VITEK (USA) et réalisé avec le matériau copolymère butadiène-styrène tel que celui vendu sous la dénomination commerciale K-Resin.

Il est également possible et, lorsque la sonde de capture est très courte (en particulier avec moins de 11 nucléotides), il devient même nécessaire, de mettre en oeuvre des moyens permettant d'améliorer la fixation de la sonde de capture sur le support solide. Comme indiqué précédemment, on peut par exemple lier la sonde de capture, de façon covalente, selon des méthodes connues en soi, à une protéine qui se fixe elle-même de façon passive sur le support (réalisé avec les matériaux déjà cités). Bien entendu, la protéine favorisant la fixation de la sonde de capture ne doit pas interférer avec la détection ; par exemple, lorsque la sonde de détection est marquée avec une enzyme, la protéine fixée sur le support solide ne doit pas avoir d'activité enzymatique interférente. Parmi les protéines que l'on peut utiliser pour la fixation passive indirecte de la sonde de capture sur le support, on peut citer l'albumine de mammifère (par exemple l'albumine bovine) ou une toxine protéique bactérienne telle que la toxine tétanique. De préférence, on choisira une protéine et/ou une méthode de couplage permettant de fixer une ou deux molécules de la sonde de capture par molécule de protéine.

Le couplage entre l'oligo-nucléotide et la protéine peut être réalisé par exemple par l'intermédiaire d'un bras alkylène ayant 3 à 12 atomes de carbone, qui peut être ajouté à l'extrémité 5' de l'oligo-nucléotide constituant la sonde de capture, lors de la synthèse automatique. Ce bras porte une fonction amine primaire qui, après activation, grâce à un agent de couplage homobifonctionnel comme le DITC(phénylème-1 ,4-diisothiocyanate), le DSS(disuccinimidyl subérate) ou le DIBS(acide 4,4'-diisothiocyano stilbène 2,2'-disulfonique), permet le greffage sur les fonctions NH₂ portées par les lysines de l'albumine. Le conjugué est purifié par chromatographie liquide haute performance sur une colonne échangeuse d'ions. On a constaté que la fixation passive de la sonde de capture par l'intermédiaire d'une protéine, comme indiqué ci-dessus, est très avantageuse, notamment car elle fournit des résultats ayant un degré élevé de reproductibilité.

La sonde de détection peut elle-même être une sonde courte, contenant de 9 à 30 nucléotides. Toutefois, pour assurer la spécificité désirée, la sonde de détection contient de préférence au moins 15 nucléotides, par exemple de 15 à 30 nucléotides, lorsque la sonde de capture est très courte et contient en particulier moins de 15 nucléotides. La sonde de détection est couplée de façon covalente à un marqueur non radioactif, par exemple une enzyme. Le couplage covalent est effectué selon les méthodes connues, en particulier par la méthode décrite ci-dessus pour le couplage entre l'oligonucléotide constituant la sonde de capture et une protéine. On peut utiliser comme marqueur enzymatique notamment une peroxydase telle que la peroxydase de raifort, une phosphatase alcaline, une phosphatase acide, la bêta-galactosidase, la glucose oxydase, etc.

Bien entendu, pour construire les sondes de capture et de détection, il est nécessaire de connaître la séquence ou une partie de la séquence de la cible, ou de connaître la séquence ou une partie de la séquence de la protéine codée par la cible. On peut alors synthétiser les oligo-nucléotides constituant les sondes avec un appareil de synthèse automatique d'ADN tel que les modèles 380 et 381 commercialisés par APPLIED BIOSYSTEMS.

Les sondes de capture et/ou de détection peuvent être constituées d'ADN ou d'ARN.

En outre, les sondes utilisées selon l'invention peuvent contenir ou être constituées par des alpha-nucléotides analogues des nucléotides naturels ; voir notamment le brevet français 2 607 507. Les alpha-nucléotides peuvent être obtenus sur synthétiseurs automatiques. Ils sont dérivables aux extrémités 3' ou 5' et se couplent aux protéines de la même manière que les bêta-oligo-nucléotides. Ils présentent l'avantage d'une bonne stabilité vis-à-vis des nucléases (voir THUONG N.T. et al., Proc. Nat. Acad. Sci. USA, 84,5129 (1987). Les conjugués alpha-oligonucléotide-enzymes sont plus stables en solution que les analogues bêta et permettent des conservations plus longues à des dilutions plus faibles.

L'acide nucléique à détecter (cible) peut être de l'ADN double brin, de l'ADN simple brin, un hybride ADN-ARN ou bien de l'ARN (ribosomal ou messager). Bien entendu, dans le cas d'une cible nucléique à double brin, ou d'un hybride ADN-ARN, il convient de procéder à sa dénaturation avant la mise en oeuvre du procédé de détection par la technique d'hybridation sandwich. L'ADN double brin peut lui-même être obtenu après extraction, selon les méthodes connues, des acides nucléiques d'un échantillon à examiner. Cette extraction peut être faite par protéolyse de cellules suivie d'une extraction au phénol-chloroforme (voir par exemple MANIATIS et Coll., "Molecular Cloning, A Laboratory Manual", Cold Spring Harbor Laboratory, New-York (1982), ou plus directement par traitement alcalin en présence de détergent et/ou par traitement aux ultra-sons, ou par tout autre procédé libérant les acides nucléiques en phase liquide. L'échantillon à traiter peut contenir un mélange de cellules, bactéries, levures, virus ou autre micro-organismes. L'ADN double brin ainsi obtenu peut être utilisé directement, après dénaturation selon les techniques connues, ou bien peut être utilisé comme matrice pour l'amplification d'acides nucléiques afin d'obtenir des quantités suffisantes pour permettre la détection.

Pour obtenir la forme simple brin à partir d'un hybride ARN-ADN ou d'un ADN double brin, on peut opérer par dénaturation physique, chimique ou enzymatique. Une des méthodes physiques de dénaturation correspond à la séparation des deux brins en chauffant l'hybride jusqu'à ce qu'il soit totalement dénaturé, par exemple à une température de 80 à 105°C pendant quelques secondes à quelques minutes. La méthode chimique de dénaturation consiste par exemple à mettre en contact l'échantillon double brin avec une solution 0,2M d'hydroxyde de sodium pendant 10 minutes, ce traitement étant suivi d'une neutralisation par l'acide acétique à la même concentration (0,2M), jusqu'à obtention d'un pH compris entre 6 et 8. Une méthode enzymatique de dénaturation consiste en l'utilisation d'une hélicase. Les techniques d'utilisation des hélicases sont décrites notamment par RADDING, Ann. Rev. Genetics, 16, 405-437 (1982). D'autres enzymes ayant la même fonction sont utilisables dans le même but.

Le procédé de l'invention est de préférence effectué selon la méthode simultanée.

Cette méthode comprend :
- la fixation passive de la sonde de capture oligonucléotidique sur le support solide, directement ou par l'intermédiaire d'une protéine, comme indiqué précédemment ; on opère par mise en contact du support avec une solution aqueuse contenant la sonde ;
- le rinçage du support et le séchage éventuel pour sa conservation ;
- la mise en contact simultanée du support ainsi obtenu avec une solution contenant l'échantillon à analyser et avec une solution contenant la sonde de détection oligonucléotidique marquée, l'échantillon et la sonde de détection pouvant être ajoutés sous forme de mélange ou séparément ;
- l'incubation du mélange obtenu ;
- le rinçage du support pour éliminer les constituants non fixés sur le support par hybridation ;
- et la détection qualitative ou quantitative à l'aide d'une réaction de révélation du marqueur fixé sur le support, par exemple par colorimétrie, fluorescence ou luminescence.

On va donner ci-après un mode de réalisation particulier, non limitatif, de la mise en oeuvre du procédé de l'invention.

### 1. Fixation de la sonde de capture

La fixation de la sonde de capture est obtenue en déposant par exemple 100 µl dans chaque puits d'une plaque de microtitration d'une solution contenant de 0,1 µg/ml à 10 µg/ml d'oligonucléotides de préférence 1 µg/ml, sous la forme d'une solution dans un tampon PBS 3X (0,45 M NaCl, 0,15 M phosphate de sodium, pH 7,0).

On laisse la plaque en contact avec la solution de sondes de capture pendant au moins 2 heures à 37°C, puis on la lave avec une solution de PBS 1X (NaCl 150 mM, tampon phosphate de sodium 50 mM, pH 7,0, contenant 0,5% de Tween^{®} 20).

### 2. Incubation simultanée des échantillons et de la sonde de détection marquée

Les échantillons (cible) sont déposés sur la plaque après avoir subi si nécessaire le traitement de dénaturation ; sous la forme d'une solution dans le tampon PBS 3X contenant de l'ADN hétérologue, par exemple de l'ADN de sperme de saumon à 10µg/ml.

La sonde marquée est diluée dans le tampon précédent à une concentration de 20 à 100 µg/ml. On dépose 50 µl de cette solution dans le puits contenant la cible.

La plaque de microtitration est alors incubée pendant un temps pouvant varier par exemple entre 30 et 120 minutes à la température choisie, par exemple 37°C ou moins longtemps si une faible sensibilité est requise.

### 3. Lavage

On effectue par exemple le lavage avec du tampon PBS 1X contenant 0,5 % Tween^{®} 20 (Merk 82 21 84).

### 4. Révélation enzymatique

La révélation est effectuée avec le substrat correspondant à l'enzyme utilisée. Par exemple, on peut ajouter de l'orthophénylène diamine (OPD) dans le cas où le marqueur est la peroxydase de raifort du paranitrophénylphosphate (PNPP) ou du méthyl-5 umbelliferyl phosphate (MUP) dans le cas où la marqueur est la phosphatase alcaline.

Le temps de révélation du substrat est compris généralement entre 1 et 30 minutes après quoi la réaction est stoppée par addition de NaOH 1N dans le cas de la phosphatase alcaline, ou de H₂SO₄ 1N dans le cas de la péroxydase de raifort.

On effectue la lecture sur un lecteur de microplaques, à 405 nm pour le PNPP, à 492 nm pour l'OPD et en fluorescence pour le MUP (excitation 340 nm, émission 460 nm).

On revient ci-après sur les stades d'incubation et de lavage consécutif qui constituent des étapes-clés du procédé d'hybridation sandwich. Ces opérations d'incubation et de lavage sont chacune effectuées à une température constante comprise entre 20 et 60°C, et de préférence entre 25 et 40°C. L'un des avantages essentiels de l'utilisation de sondes courtes est de permettre la réalisation de tests sandwich pour la détection de cibles différentes en opérant, pour ces diverses déterminations, à une température unique prédéterminée, qui sera de préférence 37°C puisque tous les laboratoires d'analyses sont équipés d'appareils thermostatés réglés à cette température.

On sain que les hybrides d'ADN ont une température de dissociation qui dépend du nombre de bases hybridées (la température augmentant avec la taille de l'hybride) et qui dépend également de la nature des bases hybridées et, pour chaque base hybridée, de la nature des bases adjacentes.

La dissociation des hybrides se produit sur un intervalle de quelques °C et peut être facilement déterminée en spectroscopie UV : en effet, la densité optique de l'hybride est inférieure à celle des séquences monobrin correspondantes. Il est donc possible de déterminer dans chaque cas une température de demi-dissociation caractéristique de l'hybride étudié.

La connaissance de l'entropie et de l'enthalpie de réaction permet d'ailleurs de calculer (loi de Vant'Hoff) la température de demi-dissociation d'un hybride donné dans des conditions standards (par exemple dans une solution de salinité correspondant à NaCl 1M) en fonction de la concentration totale en acide nucléique dans la solution ; voir par exemple Breslauer K.J. et al, PNAS, 83, 3476 (1986); RYCHLIK W. et RHOADS R.E., Nucl. Acid. Res.17, 8543 (1989) ; et FREIER S.M. et al., PNAS 83, 9373, 1986.

En pratique, il est toujours possible de déterminer expérimentalement la température de demi-dissociation de l'hybride formé par une sonde donnée avec la cible de séquence complémentaire, par de simples expériences de routine.

La température d'hybridation du protocole sandwich (température d'incubation et/ou température de lavage) devra évidemment être choisie en- dessous de la température de demi-dissociation, faute de quoi la cible ne s'hybriderait pas avec la sonde de capture et/ou de détection et les résultats du test seraient erronés.

Il est bien entendu que les considérations présentes sur la température de demi-dissociation concernent l'hybride le moins stable (c'est-à-dire celui ayant la température de demi-dissociation la plus basse) des deux hybrides que forme la cible avec d'une part la sonde de capture et d'autre part la sonde de détection. En effet, pour que le protocole sandwich puisse fonctionner correctement, il est évidemment nécessaire dans le cas où l'on recherche un résultat positif, que ces deux hybrides soient stables à la température à laquelle on opère.

Une mutation ponctuelle entraînant un mésappariement portant sur une seule paire de bases dans l'hybride, entraîne une modification, généralement un abaissement, de la température de demi-dissociation.

Un avantage essentiel résultant de l'utilisation de sondes courtes est qu'un tel mésappariement unique peut entraîner un abaissement relativement important de la température de demi-dissociation, de l'ordre de 2 à 4°C. Cela n'est pas le cas avec les sondes longues, où un tel mésappariement unique n'entraîne d'une très faible variation de la température de demi-dissociation.

On a maintenant découvert qu'il est possible de tirer avantage de l'utilisation de sondes courtes pour effectuer des déterminations selon le protocole sandwich à une température unique prédéterminée, par exemple 37°C comme indiqué ci-dessus.

Dans ce qui suit, on va raisonner à titre d'exemple sur le cas, généralement préféré, où c'est la sonde capture qui donne avec la cible recherchée un hybride moins stable que l'hybride cible-sonde de détection. Mais il est évident que l'on peut aussi adopter un système où inversement, c'est l'hybride sonde de détection-cible qui est le moins stable (il suffirait de remplacer alors dans ce qui suit l'expression "sonde de capture" par "sonde donnant avec la cible l'hybride le moins stable" pour adapter le raisonnement au cas le plus général).

Il faut bien entendu opérer avec une sonde de capture (et une sonde de détection) suffisamment longue pour que la température de demi-dissociation soit supérieure à la température choisie. Si l'on veut réaliser un test très sensible (en particulier un test sensible à une seule mutation), il conviendra d'utiliser une sonde de capture (et/ou une sonde de détection) ajustée, c'est-à-dire de longueur suffisamment courte et de séquence telle que la température de demi-dissociation ne soit que très peu supérieure à la température d'hybridation prédéterminée, l'écart entre les deux températures étant par exemple de 1 à 3°C, car il convient de travailler alors à la température maximum admissible pour éviter des hybridations non-spécifiques qui seraient possibles en adoptant une température trop basse par rapport à la température de dissociation de l'hybride parfaitement complémentaire. En fait, l'écart de température admissible peut être déterminé dans chaque cas par de simples expériences de routine.

Dans le cas qui vient d'être indiqué, à la température d'hybridation fixée, seule une cible parfaitement homologue de la sonde de capture restera fixée sur la sonde, tandis qu'une cible comportant une mutation ponctuelle dans la région complémentaire de la sonde ne sera pas fixée.

Dans la discussion précédente, on a surtout mis l'accent sur le choix d'une sonde de capture qui est adaptée à la température d'hybridation choisie principalement par des ajustements de la taille de la sonde. Mais il est bien évident pour les spécialistes qu'il est possible de jouer également sur le choix de la séquence de la sonde, puisque la température de demi-dissociation peut varier en fonction de la nature des bases impliquées dans la formation de l'hybride. Il est également possible de jouer sur les conditions d'hybridation, et en particulier sur la concentration saline de la solution d'incubation et/ou de la solution utilisée pour le lavage consécutif à l'incubation. On sait en effet que l'augmentation de la concentration saline entraîne une augmentation de la température de demi-dissociation.

Dans la mise en oeuvre pratique du procédé de l'invention, lorqu'on utilise une sonde de capture et/ou des conditions d'hybridation adaptées à la température prédéterminée choisie, on pourra opérer de deux façons.

Selon un premier mode d'exécution, on effectue l'étape d'incubation à ladite température prédéterminée et on effectue le lavage à ladite température ou à une température inférieure. Ce mode d'exécution est rendu possible puisqu'à la température à laquelle on opère, seule une cible parfaitement homologue de la sonde de capture a pu s'hybrider, de sorte qu'il n'y a pas de risque de fixation d'une cible non parfaitement homologue, même si on effectue le lavage à une température inférieure à la température d'incubation.

Selon un second mode d'exécution, on effectue l'étape d'incubation à une température inférieure à ladite température prédéterminée et on effectue le lavage à ladite température prédéterminée. En effet, dans ce cas, c'est la température de lavage qui sera discriminante, et tout hybride éventuellement formé, à la température d'incubation, entre la sonde de capture et une cible non parfaitement homologue ne sera plus stable à la température de lavage et ne restera donc pas fixé sur le support.

Pour la détection d'une mutation ponctuelle, on peut opérer de deux façons :
- soit la sonde de capture contient la mutation complémentaire de la mutation recherchée, auquel cas seule la cible mutée s'hybridera dans les conditions du test d'hybridation ;
- soit la sonde de capture est parfaitement homologue de la séquence complémentaire de la cible non mutée, auquel cas seules les cibles non mutées s'hybrideront.

Le procédé de l'invention est applicable notamment :
- à la détection de l'ADN ou de l'ARN messager des virus, ou de l'ARN des rétrovirus ;
- à la détermination de l'ADN, de l'ARN messager ou ribosomal et des hybrides ADN_{c} -ARN des bactéries, des parasites et des levures ;
- et à la détermination de l'ADN des vertébrés, et en particulier chez l'homme pour effectuer le typage HLA ou pour détecter des maladies génétiques (myopathie, diabète, mucoviscidose, etc...).

Les exemples suivants illustrent l'invention sans toutefois la limiter.

Dans ces exemples, on a représenté les séquences nucléoniques, selon la convention usuelle, avec l'extrémité 5' à gauche.

L'invention a également pour objet l'utilisation des sondes décrites dans les exemples pour la détection de la cible indiquée. Il faut bien comprendre que les sondes mentionnées dans les exemples doivent être considérées comme des séquences minimum pouvant être allongées (dans les limites indiquées de 30, 25 ou 20 nucléotides au maximum) par adjonction de nucléotides suplémentaires qui sont bien entendu choisis pour que la sonde soit homologue de la cible dont la séquence est ici supposée connue. Les sondes mentionnées comme étant satisfaisantes dans les exemples permettent toutes d'opérer à 37°C.

Dans les exemples pour lesquels on donne deux séquences pour la sonde de capture (ou pour la sonde de détection), cela signifie que l'on peut utiliser l'une des sondes ou les deux sondes. L'utilisation de deux sondes de capture et/ou de deux sondes de détection permet d'augmenter le signal lorsque les résultats ne sont pas satisfaisants avec une seule sonde.

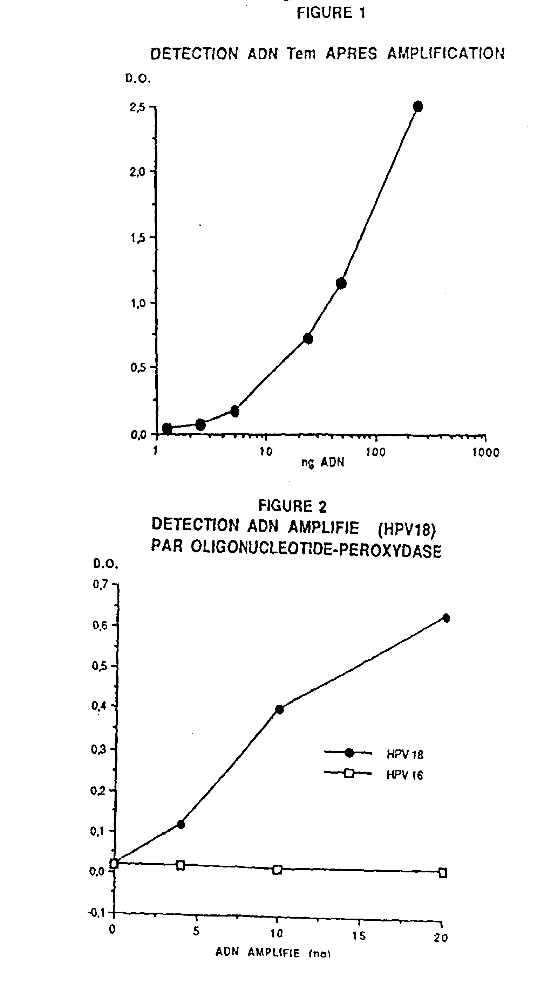

Les indications concernant les figures 1 et 2 annexées sont données respectivement aux exemples 10 et 15.

### EXEMPLE 1

Préparation du phosphoramidite-bras pour l'addition d'un bras NH₂ en 5' d'un oligonucléotide.

21,0 g (0,2 mole) d'amine-5 pentanol-1 (Aldrich 12304-8) sont dissous dans 90 ml de THF et l'on ajoute 5,3 g de Na₂CO₃. Le mélange est refroidi à - 5 ° C et l'on additionne goutte à goutte 14 g (0,067 mole) d'anhydride trifluoroacétique (Aldrich 10623-2) dissous dans 50 ml de THF en 1 h.

Le mélange est laissé 1 h sous agitation à température ambiante. Après filtration et évaporation du THF, le produit est distillé sous vide. La fraction E_{0,3} mm = 75-90° C est recueillie et purifiée sur gel de silice par un mélange CH₂Cl₂, MeOH 80 : 20.

Le produit désiré à un Rf de 0,6.. Le rendement est de 66 %. 0,7 g d'aminoalcool protégé (3,48 10⁻³ mole) sont séchés à la piridine (2 X 3 ml) avec 3 ml de N-éthyldiisopropylamine (13.92 10⁻³ mole) et dissous dans 15 ml de CH₂ Cl₂ . 1,65 g (6.96 10⁻³ mole) de cyanoéthyl-2-N,N-diisopropylchlorophosphoramidite (Aldrich 30230-9) sont additionnés sous argon à température ambiante pendant 35 mn. On ajoute 0.2 ml de MeOH et après 10 mn sous agitation, 30 ml d'acétate d'éthyle. La phase organique est lavée à froid avec 2 fois 60 ml de Na₂CO₃ 10 % puis 2 X 80 ml de NaCI saturé,séchée sur Na₂SO₄ puis évaporée à l'évaporateur rotatif.

Le produit brut est purifié sur colonne de silice dans le mélange acétate d'éthyle-CH₂Cl₂-NEt₃ (45-45-10.) La fraction recueillie (Rf - 0,75) est concentrée à l'évaporateur rotatif et conservée sous argon.

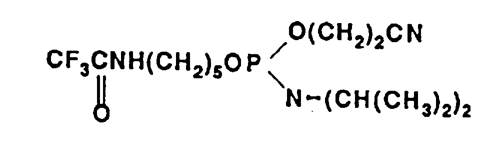

### EXEMPLE 2

### Préparation d'un conjugué oligonucléotide -sérum albumine bovine (ASB) :

L'oligonucléotide est synthétisé sur un appareil automatique APPLIED 381A en utilisant la chimie des phosphoramidites. Le bras phosphoramidite, dissout dans de l'acétonitrile anhydre à une concentration de 0.2 Molaire, est placé en position X du synthétiseur et l'addition du bras se fait selon le protocole standard en fin de synthèse. Après déprotection une nuit à 55° C dans NH₄OH 33 %, précipitation dans l'éthanol à - 20 ° C, l'oligonucléotide est séché sous vide et repris dans 1 ml d'eau. 3.10⁻⁸ mole d'oligonucléotide sont séchés sous vide et repris dans 25 µl de tampon borate de sodium 0.1 M pH 9.3. On additionne 500 µl d'une solution à 30 mg/ml de DITC (Fluka 78480) dans le DMF. Le mélange est agité 1h 30 à température ambiante avant l'addition de 3 ml d'H₂O. Après extraction de la solution par le butanol (3 x 3 ml), la phase aqueuse restante (500 µl) est séchée sous vide puis reprise par 1.10⁻⁷ mole (6,6 mg) de BSA (Pierce 30444) dans 200 µl de tampon borate. Après une nuit sous agitation à température ambiante le conjugué est purifié par échange d'ions en CLHP sur une colonne AX300 (BROWNLEE 4.6 X 100 mm) par un gradient de NaCI (Tableau 1). Le pic du conjugué est dialysé contre de l'eau (2 X 1 litre) concentré sous vide , repris par 1 ml H₂O et stocké à - 20° C.

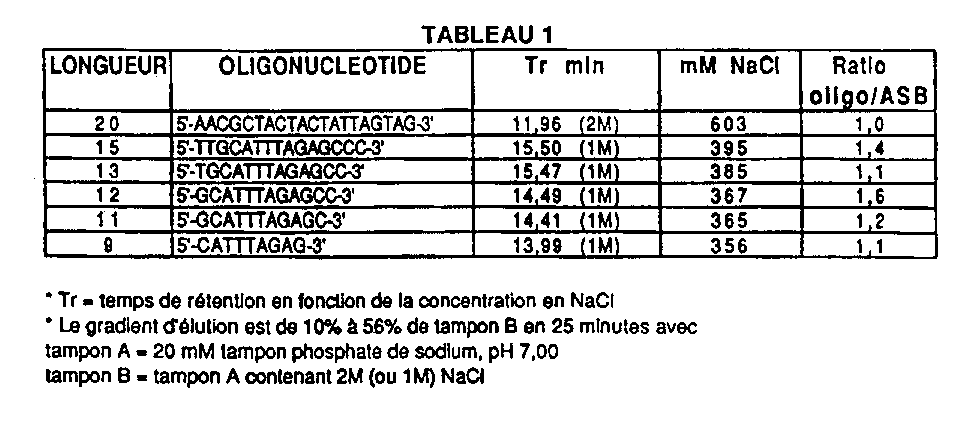

### EXEMPLE 3

Préparation d'un conjugué oligonucléotide péroxydase de raifort. Selon l'exemple 2, l'oligonucléotide activé séché sous vide est repris par 1.25 10⁻⁷ mole (5 mg de peroxydase de raifort (Boehringer 413470) dans 200 µl de tampon borate.

Le protocole de purification est identique : le conjugué est stocké à - 20°C dans un tampon 50 mM tris HCl ph 7.0, 40 % glycérol Les résultats sont présentés dans le tableau2.

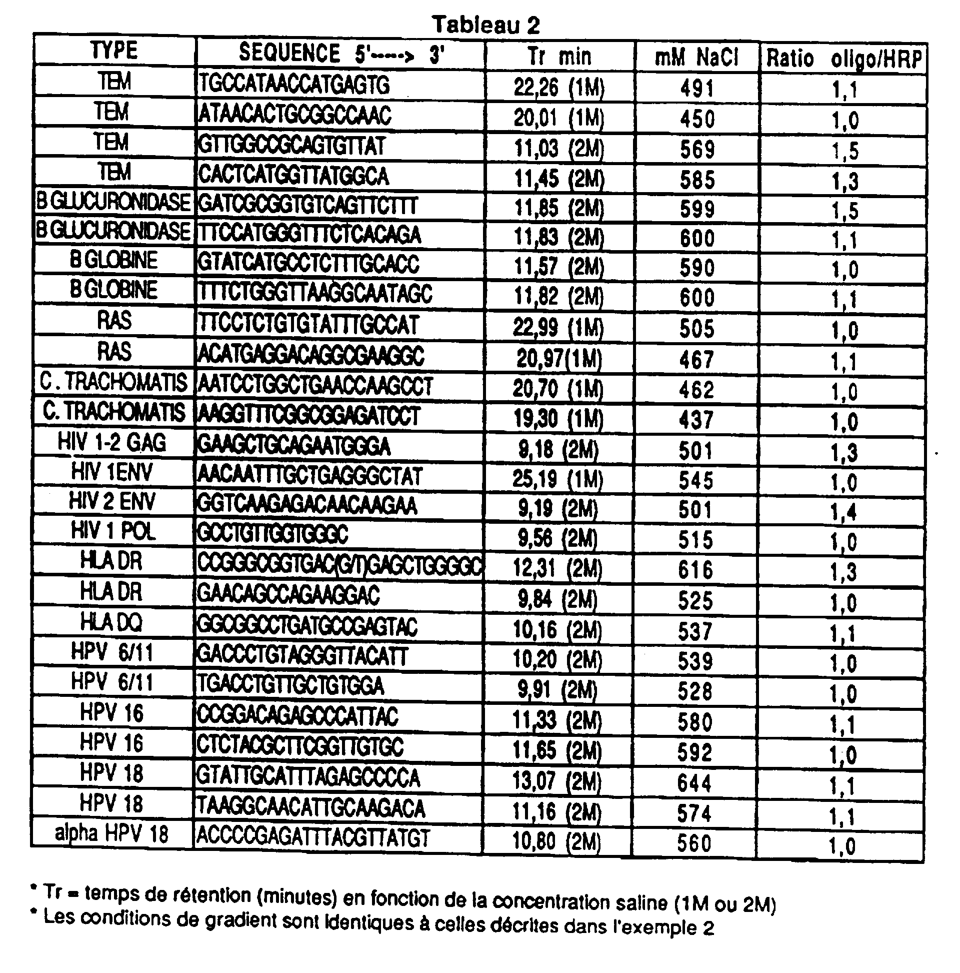

### EXEMPLE 4

Préparation d'un conjugué oligonucléotide-phosphatase alcaline. Selon l'exemple 2, l'oligonucléotide activé séché sous vide est repris par 5.7 10⁻⁸ mole (8.0 mg) de phosphatase alcaline (Boehringer 567752) dans le tampon du fabricant.

Le protocole de purification est identique (Tableau 3). Le conjugué est stocké à + 4° C dans un tampon Tris HCI 30 mM, 1M NaCI, 1 mM MgCl₂, pH 8.0.

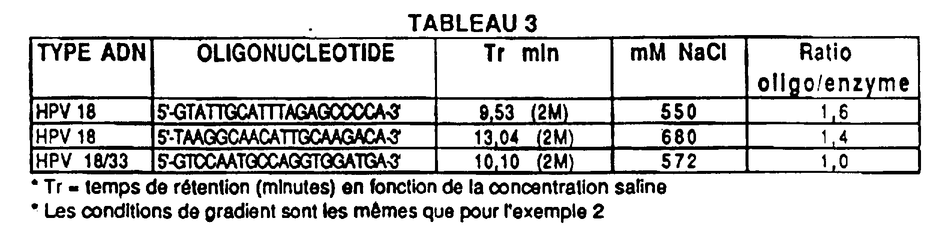

### EXEMPLE 5

Détection d'une séquence d'acide nucléique par protocole sandwich. Dans une plaque de microtitration en polystyrène (Nunc 439454) est déposée une solution de l'oligonucléotide 5'-TCAGAGGAAGAAAACGATGA-3' à 1 ng/µl (0.15µM) dans du PBS 3X (0.45 M NaCI 0.15 M phosphate de sodium ph 7.0). La plaque est incubée 2 h à 37°C.

Après 3 lavages par 300 µl de PBS Tween^{®} (0.15M NaCI, 0.05M phosphate de sodium, pH7.0, PBS Tween 20 (MERCK 822184)) 50 µl de la séquence (5'-AAGGTCAACCGGAATTTCATTTTGGGGCTCTAAATGCAATACAATGTCTTGCA ATGTTGCCTTA-3') à différentes concentrations de 100 pg/µl (5nM) à 0,01pg/µl (0.5 pM) dans du tampon PBS saumon (PBS3X + ADN de sperme de saumon 10 ug/µl (Sigma D9156)) sont ajoutés dans les puits suivi de 50 µl d'une solution du conjugué oligonucléotide-péroxydase à la concentration de 0.1 ng/µl (15nM) en oligonucléotide dans un tampon PBS cheval (PBS3X + 10 % sérum de cheval (BioMérieux 55842)). La plaque est incubée 1 h à 37° C et lavée par 3 X 300 µl de PBS Tween^{®} 100 µl de substrat OPD (ortho-phenylenediamine Cambridge Medical Biotechnology ref/456) dans un tampon OPD (0,05 M acide citrique, 0.1 M Na₂HPO_{4,} pH 4,93) à la concentration de 4 mg/ml auquel on ajoute extemporanément H₂O₂ à 30 volumes au 1/1000 sont ajoutés par puits. Après 20 minde réaction l'activité enzymatique est bloquée par 100 µl d'H₂SO₄ 1N et la lecture est effectuée sur Axia Microreader (Biomérieux) à 492 nm.

### Sensibilité (Tableau 4):

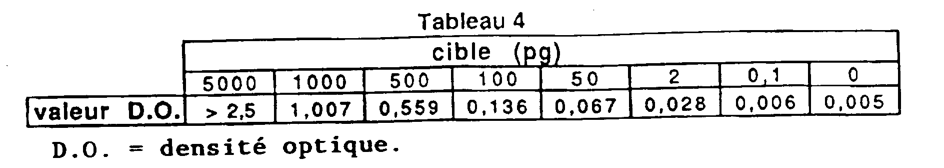

### D.O. = densité optique.

La limite de détection est de 2 pg de cible soit 10⁻¹⁷ mole.

Le système est parfaitement spécifique puisque le puits contenant l'ADN de saumon, qui est sous forme simple brin, n'est pas détecté (puits à 0 pg/µl de cible).

### EXEMPLE 6

Ainsi qu'il l'a été montré pour l'anémie falciforme ou pour les oncogènes (dans Human Genetlc diseases, a practical approach, K.E. DAVIES ed, IRL press, 1986), les mutations ponctuelles, à des positions spécifiques sur un gène, sont responsables de maladies génétiques et peuvent être détectées par hybridation de l'ADN génomique avec des sondes nucléiques. Dans certaines conditions, un mésappariement d'une base peut déstabiliser un hybride ADN-oligonucléotide. Ainsi, WALLACE R.B. et coll. (Nucl. Acid. Res., 6, 3543, 1979) détecte une mutation am-3 (A-C pour le gène muté à la place de C-G pour le gène normal) sur le gène du bactériophage φ174 par un système d'hybridation directe par filtre.

Des travaux ultérieurs ont montré que les mutations n'étaient pas équivalentes du point de vue de la déstabilisation d'un hybride. Ainsi, F. ABOUBELA et coli. (Nucl. Acid. Res., 13, 4811, 1985) trouvent que les mésappariements contenant une Guanine (GT-GG-GA) sont moins déstabilisants que les mésappariements contenant une Cytosine (CA-CC) ou un mésappariement entre 2 pyrimidines (TT-CC-TC).

S. IKUTA et coli. (Nucl. Acid. Res.,15, 797, 1987) montrent que les mésappariements GT, GA sont moins déstabilisants que AA, TT, CT, CA.

L'exemple décrit ici présente une méthode de détection d'un ADN parfaitement homologue par un protocole sandwich à 37° C. Les Oligonucléotides de capture sont fixés passivement sur la plaque . Les 5 cibles choisies sont décrites dans le tableau 5.

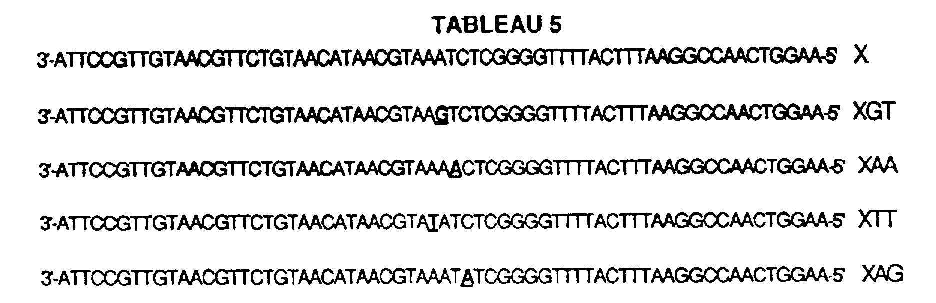

4 mutations différentes ont été introduites dans 4 séquences cibles qui diffèrent de la séquence X par 1 seule mutation.

Les mutations sont situées dans la zone 32 à 35 de la cible. Les 4 mésappariements sont de type GT (XGT), AA (XAA), TT (XTT), et AG (XAG), et on retrouve 2 mésappariements GT et AG qui sont les moins déstabilisants pour un hybride.

La sonde de détection D1 5'-TAAGGCAACATTGCAAGACA-3' est complémentaire de la région 1-20 de la cible et est commune pour toutes les cibles. Elle est marquée à l'extrémité 5' avec la péroxydase selon l'exemple 3.

La spécificité du système est amenée par les oligonucléotides de capture (Tableau 6).

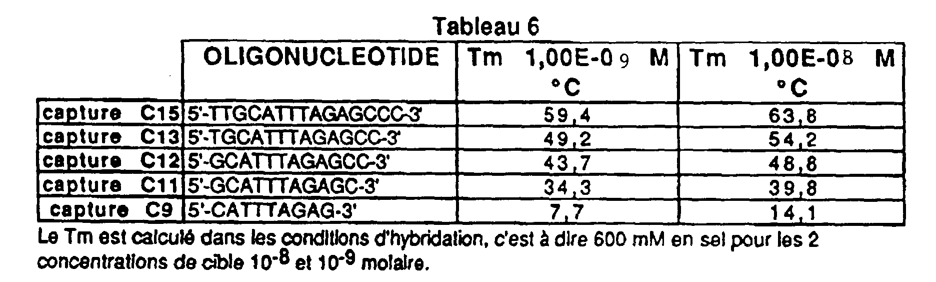

Les 5 oligonucléotides possèdent en 5' un bras C₅NH₂ pour permettre le couplage covalent sur l'albumine de sérum de boeuf (C15, C13, C12, C11, C9).

Sauf indications contraires, le protocole sandwich est identique à celui décrit dans l'exemple 5.

La cible est déposée à 10⁻⁹ mole/l soit 20 pg/µl (Tableau 7) ou 10⁻⁸ mole/1 soit 200 pg/µl (Tableau 8).

**Tableau 7**

| **CIBLE (1,00E-09 M)** | | | | | |
|---|---|---|---|---|---|
| | **X** | **XGT** | **XAA** | **XTT** | **XAG** |
| C 15 | 2,390 | 0,667 | 0,061 | 0,137 | 0,234 |
| C 13 | 0,405 | 0,030 | 0,005 | 0,008 | 0,018 |
| C 12 | 0,271 | 0,015 | 0,003 | 0,005 | 0,007 |
| C 11 | 0,100 | 0,004 | 0,004 | 0,005 | 0,005 |
| C 9 | 0,005 | 0,004 | 0,005 | 0,005 | 0,005 |
| | valeur de D.O. à 492 nm | | | | |

**Tableau 8**

| **CIBLE (1,00E-08 M)** | | | | | |
|---|---|---|---|---|---|
| | **X** | **XGT** | **XAA** | **XTT** | **XAG** |
| C 15 | >2,5 | >2,5 | 0,211 | 0,973 | 1,328 |
| C 13 | 2,320 | 0,198 | 0,010 | 0,060 | 0,081 |
| C 12 | 1,500 | 0,095 | 0,005 | 0,028 | 0,032 |
| C 11 | 0,610 | 0,014 | 0,003 | 0,014 | 0,008 |
| C 9 | 0,002 | 0,004 | 0,003 | 0,007 | 0,003 |
| | valeur de D.O. à 492 nm | | | | |

L'hybride formé avec la cible contenant un mésappariement de type GT est le moins déstabilisé donc le plus difficile à détecter. Le ratio signal X/signal XGT peut donc servir de référence pour mesurer les performances du système (Tableau 9) :

**Tableau 9**

| | **RATIO X/XGT** | |
|---|---|---|
| | 1,00E-08 | 1,00E-09 |
| C 15 | - | 4 |
| C 13 | 12 | 14 |
| C 12 | 16 | 18 |
| C 11 | 44 | 25 |
| C 9 | - | - |

L'oligonucléotide de capture C12 permet de détecter un mésappariement quelconque sur la séquence d'acide nucléique en conservant pour tous les mésappariements un signal inférieur à 0,100 pour les 2 concentrations et un ratio X/XGT >10.

### EXEMPLE 7

Méthode de détection d'un ADN parfaitement homologue à 37°C. Les oligonucléotides de capture sont couplés à l'ASB et fixés passivement sur la plaque.

Le protocole sandwich est identique à celui de l'exemple 5.

Les oligonucléotides de capture sont couplés à l'ASB selon l'exemple 2.

La sonde de détection est la même que pour l'exemple 6

Les résultats sont présentés dans les Tableaux 10,11 et 12.

**Tableau 10**

| **CIBLE (1,00E-08 M)** | | | | | |
|---|---|---|---|---|---|
| | **X** | **XGT** | **XAA** | **XTT** | **XAG** |
| C 15 | > 2,5 | > 2,5 | 0,330 | 1,445 | 2,139 |
| C 13 | > 2,5 | 0,394 | 0,025 | 0,078 | 0,092 |
| C 12 | 2,319 | 0,241 | 0,016 | 0,044 | 0,056 |
| C 11 | 0,763 | 0,025 | 0,006 | 0,007 | 0,009 |
| C 9 | 0,010 | 0,007 | 0,005 | 0,007 | 0,006 |
| | valeur de D.O. à 492 nm | | | | |

**Tableau 11**

| **CIBLE (1,00E-09 M)** | | | | | |
|---|---|---|---|---|---|
| | **X** | **XGT** | **XAA** | **XTT** | **XAG** |
| C 15 | 2,421 | 1,154 | 0,080 | 0,200 | 0,364 |
| C 13 | 0,050 | 0,075 | 0,015 | 0,019 | 0,024 |
| C 12 | 0,423 | 0,043 | 0,007 | 0,010 | 0,013 |
| C 11 | 0,134 | 0,007 | 0,007 | 0,006 | 0,009 |
| C 9 | 0,006 | 0,006 | 0,007 | 0,005 | 0,009 |

**Tableau 12**

| | **RATIO X/XGT** | |
|---|---|---|
| | 1,00E-08 | 1,00E-09 |
| C 15 | - | 2 |
| C 13 | > 6 | 7 |
| C 12 | 10 | 10 |
| C 11 | 31 | 19 |
| C 9 | - | - |

L'oligonucléotide de capture C11 couplé à l'ASB permet de détecter un mésappariement quelconque sur la séquence d'acide nucléique en conservant pour tous les mésappariements un signal inférieur à 0,100 aux 2 concentrations et un ratio X/XGT > 10.

### EXEMPLE 8

Méthode de détection d'un ADN parfaitement homologue à différentes températures .

Le protocole de détection est le même que pour l'exemple 7. L'oligonucléotide de capture C11 est couplé à l'ASB et fixé passivement sur la plaque. La concentration de la cible est constante et fixée à 10⁻⁹ molaire. La température d'hybridation est variable (Tableau 13).

**Tableau 13**

| **TEMPERATURE °C** | **X** | **XGT** | **X/XGT** |
|---|---|---|---|
| 22 | 0,155 | 0,007 | 22 |
| 30 | 0,313 | 0,024 | 13 |
| 37 | 0,229 | 0,009 | 25 |
| 45 | 0,321 | 0,013 | 25 |
| 50 | 0,052 | 0,010 | 5 |

### EXEMPLE 9

Méthode de détection d'un ADN parfaitement homologue à différentes températures.

Le protocole de détection est le même que pour l'exemple 7. L'oligonucléotide de capture C13 est fixé passivement sur la plaque directement. La concentration de la cible est constante et fixée à 10⁻⁸ molaire. La température d'hybridation est variable (Tableau 14).

**Tableau 14**

| **TEMPERATURE °C** | **X** | **XGT** | **X/XGT** |
|---|---|---|---|
| 22 | > 2,5 | 0,343 | >7 |
| 30 | 2,028 | 0,337 | 6 |
| 37 | 2,281 | 0,227 | 10 |
| 45 | 1,458 | 0,134 | 11 |
| 50 | 0,653 | 0,017 | 38 |

### EXEMPLE 10

Détection spécifique de la séquence ADN ou ARN correspondant au gène de la β lactamase de E. coli (Tem).

Dans le cas où la séquence a détecter correspond à de l'ARN messager, celui-ci est obtenu soit à partir d'une purification spécifique d'ARN (MANIATIS et coll. dans Molecular Cloning, a laboratory Manual, New York, Cold Spring Harbor Laboratory, 1982), soit à partir d'une lyse directe des bactéries après addition de soude (0,2 M final) sur le culot bactérien. Le système de détection utilisé est complémentaire du brin ARN messager correspondant au gène Tem.

Ce système se compose de 2 oligonucléotides de capture. L'adsorption passive est effectuée à une concentration totale de 0.15 µM utilisé comme décrit dans l'exemple 5 .

La détection est effectuée par l'utilisation de 2 oligonucléotides marqués par la peroxydase (à la concentration totale de 15 nM). Les séquences des oligonucléotides utilisés sont les suivantes :
CAPTURE 1 = 5'-GCACTGCATAATTCTT-3'
CAPTURE 2 = 5'-TACTGTCATGCCATCC-3'

DETECTION 1 = 5'-GTTGGCCGCAGTGTTAT-3'
DETECTION 2 = 5'-CACTCATGGTTATGGCA-3'

Détection sur ARN non amplifié, purifié (Tableau 15):

**Tableau 15**

| **ARN PURIFIE** | **Valeur D.O** |
|---|---|
| 10 µg ARN spécifique | 0,100 |
| 1 µg ARN spécifique | 0,014 |
| 10 µg ARN non spécifique | 0,006 |

Détection sur ARN de colonies non purifié (Tableau 16):

**Tableau 16**

| **NOMBRE DE COLONIES** | **valeur D.O** |
|---|---|
| 1,00E+08 colonies spécifiques | 0,150 |
| 1,00E+07 colonies spécifiques | 0,045 |
| 1,00E+06 colonies spécifiques | 0,004 |
| 1,00E+05 colonies spécifiques | 0,001 |
| 1,00E+08 colonies non spécifiques | 0,001 |

La limite de détection du système sur ARN, sans amplification, est de 1 µg d'ARN total purifié et de 10⁶ - 10⁷ colonies bactériennes.

Dans le cas où la séquence à détecter correspond à de l'ADN non amplifié (plasmide pBR 322, contenant le gène de la β lactamase). Dans ce cas, la détection est effectuée directement sur l'ADN dénaturé après les conditions décrites précédemment.

Le système de détection utilisé est le suivant :
CAPTURE 1 = 5'-GGATGGCATGACAGTA-3'
CAPTURE 2 = 5'-AGAGAATTATGCAGTGC-3'

DETECTION 1= 5'-TGCCATAACCATGAGTG-3'
DETECTION 2 = 5'-ATAACACTGCGGCCAAC-3'

Les résultats sont les suivants (Tableau 17) :

**Tableau 17**

| **ADN pBR322** | **valeur D.O** |
|---|---|
| 1µg | 0,130 |
| 0,1µg | 0,012 |
| 0,01 µg | 0,002 |
| 10 µg ADN saumon | 0,002 |

La sensibilité sur ADN non amplifié est de 0,1 µg, c'est à dire 3,48.10⁻¹⁴ mole correspondant à 2.10¹⁰ molécules vraies.

Dans le cas où l'ADN à détecter correspond à de l'ADN amplifié par réaction en chaire utilisant une polymérase, les oligonucléotides utilisés pour l'amplification sont les suivants :
AMORCE 1 = 5'-ATCAGCAATAACCAGC-3'
AMORCE 2 = 5'-CCCCGAAGAACGTTTTC-3'

L'ADN à détecter est dénaturé par le protocole précédemment cité. La détection se fait en utilisant un des deux systèmes décrits dans cet exemple.

La figure 1 montre les résultats obtenus avec en abscisses la quantité d'ADN ( ng) et en ordonnée la densité optique.

La sensibilité est de l'ordre de 1 ng d'ADN obtenu après amplification en considérant que la quantité d'ADN total générée après amplification est de l'ordre de 2,0 µg.

Pour un fragment amplifié de 560 paires de bases, le seuil de détection est donc de l'ordre de 2,94.10⁻¹⁵ mole soit 1,7 10⁹ molécules vraies.

### EXEMPLE 11

Détection de la séquence correspondant au gène E7 du papillomavirus humain type 6.

Dans le cas où la séquence à détecter est de l'ADN amplifié, les séquences utilisées sont les suivantes :
AMORCE 1 = 5'-TACACTGCTGGACAACATGC-3'
AMORCE 2 = 5'-GTGCGCAGATGGGACACAC-3'

L'ADN amplifié ou non peut être détecté par le système suivant (détection brin -)
CAPTURE 1 = 5'CAGTGTACAGAAACA-3'
CAPTURE 2 = 5'-GAGTGCACAGACGGA-3'

DETECTION 1 = 5'-GACCCTGTAGGGTTACATT-3'
DETECTION 2 = 5'-TGACCTGTTGCTGTGGA-3'

### EXEMPLE 12

Détection de la séquence ADN correspondant au gène E7 du papillomavirus humain type 11.

Dans le cas où la séquence à détecter est de l'ADN amplifié, les séquences utilisées sont les suivantes :
AMORCE 1 - 5'-TACACTGCTGGACAACATGC-3'
AMORCE 2 = 5'-GTGCGCAGATGGGACACAC-3'

L'ADN amplifié ou non peut être détecté par le système suivant (Détection brin -).
CAPTURE 1 = 5'-GAGTGCACAGACGGA-3'
CAPTURE 2 = 5'-CAACTACAAGACCTTTTGC-3'

DETECTION 1 = 5'-GACCCTGTAGGGTTACATT-3'
DETECTION 2 = 5'-TGACCTGTTGCTGTGGA-3'

### EXEMPLE 13

Détection de la séquence ADN correspondant aux gènes E7 des papillomavirus humains type 6 et 11.

Dans le cas où la séquence à détecter est de l'ADN amplifié, les séquences utilisées sont les suivantes :
AMORCE 1 = 5'TACACTGCTGGACAACATGC-3'
AMORCE 2 = 5'-GTGCGCAGATGGGACACAC-3'

L'ADN amplifié ou non peut être détecté par le système suivant (Détection brin -).
CAPTURE 1 = 5'-AGACAGCTCAGAAGATGAGG-3'
CAPTURE 2 = 5'CAGCAACGT(T/C)CGACTGGTTG-3'

DETECTION 1 = 5'-GACCCTGTAGGGTTACATT-3'
DETECTION 2 = 5'-TGACCTGTTGCTGTGGA-3'

### EXEMPLE 14

Détection de la séquence ADN correspondant au gène E7 du papillomavirus humain type 16.

Dans le cas où la séquence à détecter est de l'ADN amplifié, les séquences utilisées sont les suivantes :
AMORCE 1 = 5'-CCCAGCTGTAATCATGCATGGAGA-3'
AMORCE 2 = 5'-GTGTGCCCATTAACAGGTCTTCCA-3'

L'ADN amplifié ou non peut être détecté par le système suivant (Détection brin -).
CAPTURE 1 = 5'-TATATGTTAGATTTGCAACC-3'
CAPTURE 2 = 5'-GACAACTGATCTCTAC-3'

DETECTION 1 = 5'-CCGGACAGAGCCCATTAC-3'
DETECTION 2 - 5'-CTCTACGCTTCGGTTGTGC-3'

### EXEMPLE 15

Détection de la séquence ADN correspondant au gène E7 du papillomavirus humain type 18.

Dans le cas où la séquence à détecter est de l'ADN amplifié, les séquences utilisées sont les suivantes :
AMORCE 1 = 5'CGACAGGAACGACTCCAACG-3'
AMORCE 2 = 5'-GCTGGTAAATGTTGATGATTAACT-3'

L'ADN amplifié ou non peut être détecté par le système suivant (Détection brin-).
CAPTURE 1 - 5'-TCAGAGGAAGAAAACGATGA-3'
CAPTURE 2 = 5'-ATGTCACGAGCAATTAAGCG-3'

DETECTION 1= 5'-GTATTGCATTTAGAGCCCCA-3'
DETECTION 2 = 5'-TAAGGCAACATTGCAAGACA-3'

L'ADN à détecter est dénaturé par le protocole précédemment cité.

La figure 2 représente les résultats obtenus, avec en abscisses la quantité d'ADN, et en ordonnées la densité optique (on voit sur la figure 2 que seul 1'HPV18 est détecté, l'HPV 16 ne l'est pas).

La sensibilité est de l'ordre de 1 ng d'ADN.

### EXEMPLE 16

Système de détection de papillomavirus humain de type 6, 11, 16 et 18. L'exemple décrit la détection de chaque type après une amplification non spécifique, par réaction en chaire utilisant une polymérase.

Celle ci est effectuée avec un mélange de tous les oligonucléotides décrits dans les exemples 11, 12, 13, 14 et 15.

Chaque oligonucléotide est utilisé à une concentration de 0,3 µM. La capture se fait en utilisant l'ensemble des oligonucléotides de capture décrits dans les exemples 11, 12, 13, 14 et 15 (adsorption passive, protocole décrit exemple 5).

La détection se fait spécifiquement en utilisant l'un des couples d'oligonucléotides marqués à la peroxydase décrit dans les exemples 11, 12,13,14 et 15.

Les résultats et spécificité obtenus sont les suivants sans le cas où la détection est effectuée sur le 1/100^{e} du produit de l'amplification. (Tableau 18)

**Tableau 18**

| | **SYSTEME DE DETECTION** | | |
|---|---|---|---|
| **ADN AMPLIFIE** | **HPV18** | **HPV16** | **HPV6/11** |
| **HPV6** | 0,001 | 0,004 | 1,100 |
| **HPV16** | 0,002 | 0,840 | 0,008 |
| **HPV18** | 0,600 | 0,028 | 0,002 |
| **HPV6+16** | 0,001 | 0,845 | 0,480 |
| **HPV6+16+18** | 0,540 | 0,821 | 0,320 |
| **HPV6+18** | 0,580 | 0,020 | 0,687 |
| **HPV+16+18** | 0,516 | 0,740 | 0,007 |
| **HPV2 non spécifique** | 0,001 | 0,018 | 0,002 |
| **Eau : témoin non spécifique** | 0,001 | 0,014 | 0,001 |

### EXEMPLE 17

Détection de la séquence ADN correspondant au gène de la protéine majeure de la membrane externe (MOMP) de Chlamydia trachomatis. Dans le cas où la séquence à détecter est de l'ADN amplifié, les séquences utilisées sont les suivantes :
AMORCE D'AMPLIFICATION 1 = 5'-CACCATAGTAACCCATACGC-3'
AMORCE D'AMPLIFICATION 2 = 5'-GCCGCTTTGAGTTCTGCTTCC-3'

L'ADN amplifié ou non peut être détecté par le système suivant (détection brin - ).
CAPTURE 1 = 5'-GCCGCTTTGAGTTCTGCTTCC-3'
CAPTURE 2 = 5'-CTTGCAAGCTCTGCCTGTGG-3'

DETECTION 1 = 5'-AATCCTGGCTGAACCAAGCCT-3'
DETECTION 2 = 5'-AAGGTTTCGGCGGAGATCCT-3'

### EXEMPLE 18

Détection de la séquence ADN correspondant à la région gag du rétrovirus HIV 1.

Dans le cas où la séquence à détecter est de l'ADN amplifié, les séquences utilisées sont les suivantes :
AMORCE 1 = 5'-GGACATCAAGCAGCCATGC-3'
AMORCE 2 = 5'-CTAGTAGTTCCTGCTATGTC-3'

L'ADN amplifié ou non peut être détecté par le système suivant
CAPTURE 1 - 5'-AATGTTAAAAGAGAC-3'
DETECTION 1- 5'-GAAGCTGCAGAATGGGA-3'

### EXEMPLE 19

Détection de la séquence ADN correspondant à la région gag du rétrovirus HIV 2.

Dans le cas où la séquence à détecter est de l'ADN amplifié, les séquences utilisées sont les suivantes :
AMORCE 1 = 5'-GACCATCAAGCAGCCATGC-3'
AMORCE 2 = 5'-CTTGTTGTCCCTGCTATGTC-3'

L'ADN amplifié ou non peut être détecté par le système suivant
CAPTURE = 5'-CTTACCAGCGGGGCAGC-3'
DETECTION 1 = 5'-GAAGCTGCAGAATGGGA-3'

### EXEMPLE 20

Détection de la séquence ADN correspondant à la région env du rétrovirus HIV 1.

Dans le cas où la séquence à détecter est de l'ADN amplifié, les séquences utilisées sont les suivantes :
AMORCE 1 - 5'-CAGGAAGCACTATGGGCGC-3'
AMORCE 2 = 5'-GCTGCTTGATGCCCCAGAC-3'

L'ADN amplifié ou non peut être détecté par le système suivant
CAPTURE 1 = 5'-TGTCTGGTATAGTGCA-3
DETECTION 1 = 5'-AACAATTTGCTGAGGGCTAT-3''

### EXEMPLE 21

Détection de la séquence ADN correspondant à la région env du rétrovirus HIV 2.

Dans le cas où la séquence à détecter est de l'ADN amplifié, les séquences utilisées sont les suivantes :
AMORCE 1 = 5'-CAGGCAGTTCTGCAATGGG-3'
AMORCE 2 = 5'-GGTTtttCGTTCCCCAGACG-3'

L'ADN amplifié ou non peut être détecté par le système suivant
CAPTURE 1 = 5'-GCAACAGCAACAGCTGTTGGA-3'
DETECTION 1 = 5'-GGTCAAGAGACAACAAGAA-3'

### EXEMPLE 22

Détection de la séquence ADN correspondant à la région pol du rétrovirus HIV 1.

Dans le cas où la séquence à détecter est de l'ADN amplifié, les séquences utilisées sont les suivantes :
AMORCE 1 = 5'-ATTAGCAGGAAGATGGCCAG-3' AMORCE 2 = 5'-CTGCCATTTGTACTGCTGGTC-3'

L'ADN amplifié ou non peut être détecté par le système suivant
CAPTURE 1 = 5'-GACAATGGCAGCAATTTCACC-3'
DETECTION 1 = 5'-GCCTGTTGGTGGGC-3'

### EXEMPLE 23

Détection de la séquence ADN correspondant au gène de la région β glucuronidase de E coli.

Dans le cas où la séquence à détecter est de l'ADN amplifié, les séquences utilisées sont les suivantes :
AMORCE 1 = 5'-CAATACGCTCGAACGACGT-3'
AMORCE 2 = 5'-CACGGGTTGGGGTTTCTAC-3'

L'ADN amplifié ou non peut être détecté par le système suivant (détection brin -)
CAPTURE 1 = 5'-TGGCTTCTGTCAACGCTGTT-3'
CAPTURE 2 = 5'-ATGCGATCTATATCACGCTG-3'

DETECTION 1 = 5'-GATCGCGGTGTCAGTTCTTT-3'
DETECTION 2 = 5'-TTCCATGGGTTTCTCACAGA-3'

### EXEMPLE 24

Détection de la séquence ADN correspondant au gène de la région β globine humaine.

Dans le cas où la séquence à détecter est de l'ADN amplifié, les séquences utilisées sont les suivantes :
AMORCE 1 = 5'-GACTCAGAATAATCCAGCCT-3'
AMORCE 2 = 5'-TGTTTACGCAGTCTGCCTAG-3'

L'ADN amplifié ou non peut être détecté par le système suivant (détection brin -)
CAPTURE 1 = 5'-TGTTTACGCAGTCTGCCTAG-3'
CAPTURE 2 = 5'-CACATATTGACCAAATCAGG-3'

DETECTION 1 = 5'-GTATCATGCCTCTTTGCACC-3'
DETECTION 2 = 5'-TTTCTGGGTTAAGGCAATAGC-3'

### EXEMPLE 25

Détection de la séquence ADN correspondant à l'oncogéne ras Dans le cas où la séquence à détecter est de l'ADN amplifié, les séquences utilisées sont les suivantes :
AMORCE 1 = 5'-TGTTATGATGGTGAAACCTG-3'
AMORCE 2 = 5'-CTGTAGAGGTTAATATCCGCAAA-3'

L'ADN amplifié ou non peut être détecté par le système suivant (détection brin -).
CAPTURE 1 = 5'-CAGTGCCATGAGAGACCAAT-3'
CAPTURE 2 = 5'-CGACGCAGCCATGGTCGATGC -3'

DETECTION 1 = 5'-TTCCTCTGTGTATTTGCCAT-3'
DETECTION 2 = 5'-ACATGAGGACAGGCGAAGGC-3'

### EXEMPLE 26

Utilisation d'alphaoligonucléotide marqué à la péroxydase en détection. Protocole selon l'exemple 5.

Cible :

5'-TCATCGTTTTCTTCCTCTGAGTCGCTTAATTGCTCGTGACATAGAAGGTCAACCG
GAATTTCATTTTGGGGCTCTAAATGCAATACAATGTCTTGCAATG TTGCCTTA -3'

Capture :

: 5'-TCAGAGGAAGAAAACGATGA-3'

La sonde de détection utilisée est l'alpha oligonucléotide 5'-ACCCCGAGATT TAGGTTATGT-3' greffé à la peroxydase de raifort selon l'exemple 3. La limite de détection est de 5 pg de cible soit 5.10⁻¹⁷ mole (Tableau 19).

**Tableau 19**

| | **cible (pg)** | | | | | |
|---|---|---|---|---|---|---|
| | 5000 | 500 | 50 | 5 | 0,5 | 0 |
| **valeur D.O.** | >2,5 | 0,622 | 0,073 | 0,025 | 0,008 | 0,007 |

### EXEMPLE 27

Phosphatase alcaline comme enzyme de détection en colorimétrie.

Protocole selon l'exemple 5 :

La sonde de détection 5'-TAAGGCAACATTGCAAGACA-3' est couplée à la phosphatase alcaline selon l'exemple 4.

100 µl de substrat PNPP (Sigma 104-0) à la concentration de 2 mg/ml (dans un tampon diéthanolamine1,29 M; MgSO₄ 0,56 mM; NaN₃ 0,38 mM; HCI 0,012 N; pH 9,8) sont ajoutés par puits. Après 20 mn de réaction, l'activité enzymatique est bloquée par 100 µl de NaOH 1 N et la lecture est effectuée sur Axiamicroreader (BioMérieux) à 402 nm.

Capture : 5'-TCAGAGGAAGAAAACGATGA-3'

Cible : plasmide p^{BR}322 contenant le gène du PVH de type 18 à 20 µg/ml

**Tableau 20**

| | **dilution de la cible** | | | | | |
|---|---|---|---|---|---|---|
| | 100 | 500 | 1000 | 2000 | 5000 | blanc |
| **valeur D.O.** | 0,505 | 0,086 | 0,054 | 0,029 | 0,008 | 0,008 |

La limite de détection est le 1/2000 de la cible (Tableau 20), c'est à dire de l'ordre de 500 pg soit 1.8 10-¹⁶ mole.

### EXEMPLE 28

Phosphatase alcaline comme enzyme de détection en fluorescence. Le protocole est le même que pour l'exemple 27 mais l'on rajoute comme substrat 100 µl de méthyl-4 umbelliferyl phosphate (MUP, Boehringer ref. 405663) dans un tampon glycine-NaOH 100 mM, MgCl₂ 1 mM, ZnCl₂ 0.1 mM, NaN₃ 0.5 g/l, pH 10.3, à la concentration de 80 µg/ml.

La réaction est bloquée après 20 minutes par 100 µl de KH₂PO₄ 0,5M, pH 10,4, 10 mM EDTA et lue au fluorimètre (excitation 340 nm, émission 460 nm) (Tableau 21)

**Tableau 21**

| | **dilution de la cible** | | | | | |
|---|---|---|---|---|---|---|
| | 100 | 500 | 1000 | 2000 | 5000 | blanc |
| **unités fluorescence** | 643 | 167 | 94 | 82 | 66 | 47 |

### EXEMPLE 29

Détection du virus HIV 1
a) Région NEF
   Pour l'amplification, on utilise les séquences suivantes 
AMORCE 1 | 5'-CATTGGTCTTAAAGGTACCTG-3'
AMORCE 2 | 5'-AAGATGGGTGGCAAITGGTC-3'
   Les sondes de capture et de détection sont les suivantes : 
CAPTURE | 5'-GAGGAGGTIGGTTTTCCAGTCA-3'
 |
DETECTION | 5'-GGATGGCCTICTITAAGGGAAAGAATG-3'
b) Région VPR
   Pour l'amplification, on utilise les séquences suivantes : 
AMORCE 1 | 5'-TGGAACAAGCCCCAGIAGACC-3'
AMORCE 2 | 5'-TGCTATGTIGACACCCAATTCTG-3'
 Les sondes de capture et de détection sont les suivantes : 
CAPTURE | 5'-TATGAAACTTATGGGGATAC-3'
 |
DETECTION | 5'-GAAGCTGTTAGACATTTTCCTAG-3'
c) Région POL
   Les sondes de capture et de détection sont les suivantes :

CAPTURE | 5'-TGAACAAGTAGATAAATTAG-3'
 |
DETECTION | 5'-GGAATCAGIAAAGTACTATT-3'

## Revendications

1. Procédé de détection d'une séquence nucléotidique cible simple brin dans un échantillon la contenant ou susceptible de la contenir, selon la technique d'hybridation sandwich, caractérisé par le fait qu'il comporte les étapes consistant :
(a) à faire incuber l'échantillon :
(i) avec une sonde de capture fixée sur un support solide, ledit support solide étant réalisé en un matériau hydrophobe et ladite sonde de capture étant constituée par un oligonucléotide capable de se fixer par adsorption sur ledit support solide ou par un oligonucléotide lié à une protéine par covalence, ledit oligonucléotide étant fixé par adsorption sur ledit support solide soit directement soit par l'intermédiaire de ladite protéine lorsqu'elle est présente, ledit oligonucléotide ayant de 9 à 30 nucléotides, étant entendu que lorsque ladite sonde de capture est constituée par un oligonucléotide non lié à une protéine, l'oligonucléotide comporte au moins 11 nucléotides, et
(ii) avec une sonde de détection marquée avec un marqueur non radioactif, les sondes de capture et de détection étant capables d'hybridation, respectivement, avec deux régions non chevauchantes de la séquence nucléotidique-cible recherchée, puis
(b) à laver le support solide pour éliminer les réactifs non fixés par hybridation, et
(c) à détecter la séquence cible à l'aide d'une réaction de révélation du marqueur fixé sur le support.

2. Procédé selon la revendication 1, caractérisé par le fait que ledit matériau hydrophobe est un polymère hydrophobe.

3. Procédé selon la revendication 2, caractérisé par le fait que ledit polymère est un polymère ou copolymère de styrène.

4. Procédé selon la revendication 1, caractérisé par le fait que ledit oligonucléotide contient au plus 25, et en particulier au plus 20 nucléotides.

5. Procédé selon l'une quelconque des revendications précédentes, caractérisé par le fait que l'on opère à une température pré-déterminée, et que l'on choisit des sondes de capture et de détection de taille suffisamment longue et de séquence telle que l'hybride que chaque sonde forme avec la cible recherchée soit stable à ladite température, dans les conditions d'hybridation utilisées.

6. Procédé selon la revendication 5, caractérisé par le fait que la sonde de capture et/ou la sonde de détection est suffisamment courte et de séquence telle que seule la présence d'une séquence complémentaire parfaitement homologue dans la cible permette de former avec ladite sonde suffisamment courte un hybride stable à ladite température dans les conditions d'hybridation utilisées.

7. Procédé selon la revendication 5 ou 6, caractérisé par le fait que l'on effectue l'étape d'incubation à ladite température prédéterminée et que l'on effectue le lavage à ladite température ou à une température inférieure.

8. Procédé selon la revendication 5 ou 6, caractérisé par le fait que l'on effectue l'étape d'incubation à une température inférieure à ladite température prédéterminée, et que l'on effectue le lavage à ladite température prédéterminée.

9. Procédé selon l'une quelconque des revendications 5 à 8, caractérisé par le fait que ladite température prédéterminée, est comprise entre 20 et 60°C, notamment entre 25 et 40°C, et est en particulier égale à 37°C.

10. Procédé selon l'une quelconque des revendications précédentes, caractérisé par le fait que la sonde de détection contient au moins 9 nucléotides.

11. Procédé selon la revendication 10, caractérisé par le fait que la sonde de détection contient au moins 15 nucléotides.

12. Procédé selon l'une quelconque des revendications précédentes, caractérisé par le fait que l'hybride formé par la sonde de capture et la cible recherchée est moins stable que l'hybride formé par ladite cible avec la sonde de détection.

13. Procédé selon l'une quelconque des revendications précédentes, caractérisé par le fait que la sonde de détection est plus longue, en nombre de nucléotides, que la sonde de capture.

14. Procédé selon l'une quelconque des revendications précédentes, caractérisé par le fait que la sonde de capture et/ou la sonde de détection contient ou est constituée par des alpha-D-nucléotides.

15. Procédé selon l'une quelconque des revendications précédentes caractérisé par le fait que ladite protéine est une albumine de mammifère ou une toxine protéique bactérienne.

16. Procédé selon l'une quelconque des revendications précédentes, caractérisé par le fait que le support est sous forme de tube, de bille, de particules ou de cône de pipettage, ou sous forme de plaque de microtitration.

17. Procédé selon l'une quelconque des revendications précédentes, caractérisé par le fait que l'on opère selon la technique d'hybridation sandwich simultanée.

18. Procédé selon l'une quelconque des revendications précédentes, caractérisé par le fait que ledit oligonucléotide est soit une séquence parfaitement homologue de la séquence complémentaire de la cible, soit une séquence contenant une mutation ponctuelle par rapport à la séquence complémentaire de la cible.

19. Procédé selon l'une quelconque des revendications précédentes, caractérisé par le fait que la cible est la séquence d'ADN du papillomavirus humain HPV 18 et que la sonde de capture contient la séquence

TCAGAGGAAGAAAACGATGA

20. Procédé selon la revendication 21, caractérisé par le fait que la sonde de détection contient la séquence :

TAAGGCAACATTGCAAGACA

21. Procédé selon l'une quelconque des revendications 1 à 18, caractérisé par le fait que la cible est une séquence ARN messager correspondant au gène de la bêta-lactamase de E. Coli (Tem) et que la sonde de capture contient la séquence suivante : 5'-GCACTGCATAATTCTT-3' ou

5'-TACTGTCATGCCATCC-3'

22. Procédé selon la revendication précédente, caractérisé par le fait que la sonde de détection contient la séquence suivante : 5'-GTTGGCCGCAGTGTTAT-3' ou

5'-CACTCATGGTTATGGCA-3'

23. Procédé selon l'une quelconque des revendications 1 à 18. caractérisé par le fait que la cible est une séquence d'ADN contenant le gène de la bêta-lactmase de F. coli et que la sonde de capture contient la séquence suivante : 5'-GGATGGCATGACAGTA-3' ou

5'-AGAGAATTATGCAGTGC-3'

24. Procédé selon la revendication précédente, caractérisé par le fait que la sonde de détection contient la séquence suivante : 5'-TGCCATAACCATGAGTG-3' ou

5'-ATAACACTGCGGCCAAC-3'

25. Procédé selon l'une quelconque des revendications 1 à 18, caractérisé par le fait que la cible est la séquence ADN correspondant au gène E7 du papillomavirus humain type 6 et que la sonde de capture contient la séquence suivante : 5'-CAGTGTACAGAAACA-3' ou

5'-GAGTGCACAGACGGA-3'

26. Procédé selon la revendication précédente, caractérisé par le fait que la sonde de détection contient la séquence suivante : 5'-GACCCTGTAGGGTTACATT-3' ou

5'-TGACCTGTTGCTGTGGA-3'

27. Procédé selon l'une quelconque des revendications 1 à 18, caractérisé par le fait que la cible est la séquence ADN correspondant au gène E7 du papillomavirus humain type 11 et que la sonde de capture contient la séquence suivante : 5'-GAGTGCACAGACGGA-3' ou

5'-CAACTACAAGACCTTTTGC-3'

28. Procédé selon la revendication précédente, caractérisé par le fait que la sonde de détection contient la séquence suivante : 5'-GACCCTGTAGGGTTACATT-3' ou

5'-TGACCTGTTGCTGTGGA-3'

29. Procédé selon l'une quelconque des revendications 1 à 18, caractérisé par le fait que la cible est la séquence ADN correspondant aux gènes E7 des papillomavirus humains types 6 ou il, et que la sonde de capture contient la séquence suivante : 5'-AGACAGCTCAGAAGATGAGG-3' ou

5'-CAGCAACGT(T/C)CGACTGGTTG-3'

30. Procédé selon la revendication précédente, caractérisé par le fait que la sonde de détection contient la séquence suivante : 5'-GACCCTGTAGGGTTACATT-3' ou

5'-TGACCTGTTGCTGTGGA-3'

31. Procédé selon l'une quelconque des revendications 1 à 18, caractérisé par le fait que la cible est la séquence ADN correspondant au gène E7 du papillomavirus humain type 16 et que la sonde de capture contient la séquence suivante : 5'-TATATGTTAGATTTGCAACC-3' ou

5'-GACAACTGATCTCTAC-3'

32. Procédé selon la revendication précédente, caractérisé par le fait que la sonde de détection contient la séquence suivante : 5'-CTCTACGCTTCGGTTGTGC-3' ou

5'-CCGGACAGAGCCCATTAC-3'

33. Procédé selon l'une quelconque des revendications 1 à 18, caractérisé par le fait que la cible est la séquence ADN correspondant au gène E7 du papillomavirus humain type 18 et que la sonde de capture contient la séquence suivante : 5'-TCAGAGGAAGAAAACGATGA-3' ou

5'-ATGTCACGAGCAATTAAGCG-3'

34. Procédé selon la revendication précédente, caractérisé par le fait que la sonde de détection contient la séquence suivante : 5'-GTATTGCATTTAGAGCCCCA-3' ou

5'-TAAGGCAACATTGCAAGACA-3'

35. Procédé selon l'une quelconque des revendications 1 à 18, caractérisé par le fait que la cible est la séquence ADN correspondant au papillomavirus humain de type 18 et que la sonde de capture contient la séquence suivante :

5'-TCAGAGGAAGAAAACGATGA-3'

36. Procédé selon la revendication précédente, caractérisé par le fait que la sonde de détection contient la séquence suivante :

5'-TAAGGCAACATTGCAAGACA-3'

37. Procédé selon l'une quelconque des revendications 1 à 18, caractérisé par le fait que la cible est la séquence ADN correspondant au gène de la protéine majeure de la membrane externe (MOMP) de Chlamydia trachomatis et que la sonde de capture contient la séquence suivante : 5'-GCCGCTTTGAGTTCTGCTTCC-3' ou

5'-CTTGCAAGCTCTGCCTGTGG-3'

38. Procédé selon la revendication précédente, caractérisé par le fait que la sonde de détection contient la séquence suivante : 5'-AATCCTGGCTGAACCAAGCCT-3' ou

5'-AAGGTTTCGGCGGAGATCCT-3'

39. Procédé selon l'une quelconque des revendications 1 à caractérisé par le fait que la cible est une séquence correspondant, sous forme d'ADN, à la région gag du rétrovirus HIV1 et que la sonde de capture contient la séquence suivante :

5'-AATGTTAAAAGAGAC-3'

40. Procédé selon la revendication précédente, caractérisé par le fait que la sonde de détection contient la séquence suivante :

5'-GAAGCTGCAGAATGGGA-3'

41. Procédé selon l'une quelconque des revendications 1 à 18, caractérisé par le fait que la cible est une séquence correspondant, sous forme d'ADN, la région gag du rétrovirus HIV2 et que la sonde de capture contient la séquence suivante :

5'-CTTACCAGCGGGCAGC-3'

42. Procédé selon la revendication précédente, caractérisé par le fait que la sonde de détection contient la séquence suivante :

5'-GAAGCTGCAGAATGGGA-3'

43. Procédé selon l'une quelconque des revendications 1 à 18, caractérisé par le fait que la cible est une séquence correspondant, sous forme d'ADN, à la région env du rétrovirus HIV1 et que la sonde de capture contient la séquence suivante :

5'-TGTCTGGTATAGTGCA-3'

44. Procédé selon la revendication précédente, caractérisé par le fait que la sonde de détection contient la séquence suivante :

5'-AACAATTTGCTGAGGGCTAT-3'

45. Procédé selon l'une quelconque des revendications 1 à 18, caractérisé par le fait que la cible est une séquence correspondant, sous forme d'ADN, à la région env du rétrovirus HIV2 et que la sonde de capture contient la séquence suivante :

5'-GCAACAGCAACAGCTGTTGGA-3'

46. Procédé selon la revendication précédente, caractérisé par le fait que la sonde de détection contient la séquence suivante :

5'-GGTCAAGAGACAACAAGAA-3'

47. Procédé selon l'une quelconque des revendications 1 à 18, caractérisé par le fait que la cible est une séquence correspondant, sous forme d'ADN, à la région pol du rétrovirus HIV1 et que la sonde de capture contient la séquence suivante :

5'-GACAATGGCAGCAATTTCACC-3'

48. Procédé selon la revendication précédente, caractérisé par le fait que la sonde de détections contient la séquence suivante :

5'-GCCTGTTGGTGGGC-3'

49. Procédé selon l'une quelconque des revendications 1 à 18, caractérisé par le fait que la cible est la séquence ADN correspondant au gène de la région, bêta-glucuronidase de E. Coli. et que la sonde de capture contient la séquence suivante : 5'-TGGCTTCTGTCAACGCTGTT-3' ou

5'-ATGCGATCTATATCACGCTG-3'

50. Procédé selon la revendication précédente, caractérisé par le fait que la sonde de détection contient la séquence suivante : 5'-GATCGCGGTGTCAGTTCTTT-3' ou

5'-TTCCATGGGTTTCTCACAGA-3'

51. Procédé selon l'une quelconque des revendications 1 à 18, caractérisé par le fait que la cible est la séquence ADN correspondant au gène de la région bêta-globine humaine et que la sonde de capture contient la séquence suivante : 5'-TGTTTACGCAGTCTGCCTAG-3' ou

5'-CACATATTGACCAAATCAGG-3'

52. Procédé selon la revendication précédente, caractérisé par le fait que la sonde de détection contient la séquence suivante : 5'-GTATCATGCCTCTTTGCACC-3' ou

5'-TTTCTGGGTTAAGGCAATAGC-3'

53. Procédé selon l'une quelconque des revendications 1 à 18, caractérisé par le fait que la cible est la séquence ADN correspondant à l'oncogène ras et que la sonde de capture contient la séquence suivante : 5'-CAGTGCCATGAGAGACCAAT-3' ou

5'-CGACGCAGCCATGGTCGATGC -3'

54. Procédé selon la revendication précédente, caractérisé par le fait que la sonde de détection contient la séquence suivante : 5'-TTCCTCTGTGTATTTGCCAT-3' ou

5'-ACATGAGGACAGGCGAAGGC-3'

55. Procédé selon l'une quelconque des revendications 1 à 18, caractérisé par le fait que la cible est la séquence ADN de HPV type 18 et que la sonde de capture contient la séquence suivante :

5'-TCAGAGGAAGAAAACGATGA-3'

56. Procédé selon la revendication précédente, caractérisé par le fait que la sonde de détection contient la séquence (alpha-nucléotidique) suivante :

alpha oligonucléotide 5'-ACCCCGAGATT

57. Procédé selon l'une quelconque des revendications 1 à 18, caractérisé par le fait que la cible est une séquence correspondant à la région NEF du rétrovirus BIV1, et que la sonde de capture contient la séquence :

5'-GAGGAGGTIGGTTTTCCAGTCA-3'

58. Procédé selon la revendication précédente, caractérisé par le fait que la sonde de détection contient la séquence :

5'-GGATGGCCTICTITAAGGGAAAGAATG-3'

59. Procédé selon l'une quelconque des revendications 1 à 18, caractérisé par le fait que la cible est une séquence correspondant à la région VPR du rétrovirus HIV1, et que la sonde de capture contient la séquence :

5'-TATGAAACTTATGGGGATAC-3'

60. Procédé selon la revendication précédente, caractérisé par le fait que la sonde de détection contient la séquence :

5'-GAAGCTGTTAGACATTTTCCTAG-3'

61. Procédé selon l'une quelconque des revendications 1 à 18, caractérisé par le fait que la cible est une séquence correspondant à la région POL. du rétrovirus HIV1, et que la sonde de capture contient la séquence :

5'-TGAACAAGTAGATAAATTAG-3'

62. Procédé selon la revendication précédente, caractérisé par le fait que la sonde de détection contient la séquence :

5'-GGAATCAGIAAAGTACTATT-3'

## Patentansprüche

1. Verfahren zur Erkennung einer Nukleotidsequenz als einfachen Targetstrang in einer Probe, die eine solche Sequenz enthält oder dazu befähigt ist, eine solche aufzunehmen oder zu enthalten auf Basis einer Sandwich-Hybridisierungsmethode, dadurch gekennzeichnet, daß die folgenden Schritte miteingeschlossen sind:
(a) Inkubieren der Probe:
(i) mit einer auf einem festen Träger fixierten Aufnahmesonde, wobei der feste Träger aus einem hydrophoben Material gefertigt und die Aufnahmesonde aus einem Oligonukleotid besteht, das dazu befähigt ist, auf diesem festen Träger oder durch ein an ein Protein kovalent gebundenes Oligonukleotid durch Absorption fixiert zu werden und wobei dieses Oligonukleotid durch Adsorption auf diesem festem Träger entweder direkt oder mittels dieses Proteins fixiert wird, sofern letzteres vorhanden ist und wobei dieses Oligonukleotid 9-30 Nukleotide aufweist, und zwar mit der Maßgabe, daß, wenn die Aufnahmesonde aus einem nicht an ein Protein gebundenen Oligonukleotid besteht, dieses Oligonukleotid mindestens 11 Nukleotide aufweist sowie
(ii) mit einer Erkennungssonde, die mit einem nicht-radioaktiven Marker markiert ist, wobei die Aufnahme- und Erkennungssonden zur Hybridisierung befähigt sind, mit zwei nicht überlappenden Regionen der gesuchten Target-Nukleotidsequenz und schließlich
(b) Waschen des festen Trägers zur Entfernung der nicht fixierten Reaktanten durch Hybridisieren und
(c) Erkennen der Targetsequenz mittels der Erkennungsreaktion des auf dem Träger fixierten Markers.

2. Verfahren nach Anspruch 1, dadurch gekennzeichnet, daß das hydrophobe Material ein hydrophobes Polymer ist.

3. Verfahren nach Anspruch 2, dadurch gekennzeichnet, daß das Polymer ein Styrolpolymer oder Styrolcopolymer darstellt.

4. Verfahren nach Anspruch 1, dadurch gekennzeichnet, daß das Oligonukleotid höchstens 25, insbesondere höchstens 20 Nukleotide enthält.

5. Verfahren nach einem der vorstehenden Ansprüche, dadurch gekennzeichnet, daß bei einer vorbestimmten Temperatur gearbeitet wird, daß die Aufnahmesonden und die Erkennungssonden eine ausreichende Länge und eine so große Sequenz besitzen und daß das Hybrid, das jeweils durch die Sonde mit dem gesuchten Target gebildet wird, bei dieser Temperatur unter den zum Einsatz gelangenden Hybridisierungsbedingungen stabil ist.

6. Verfahren nach Anspruch 5, dadurch gekennzeichnet, daß die Aufnahmesonde und/oder die Erkennungssonde ausreichend kurz ist, und eine Sequenz von einer Größe besitzt, daß allein bei Anwesenheit einer Komplementärsequenz mit perfekter Entsprechung in dem Target die Bildung eines stabilen Hybrids bei dieser Temperatur unter den zum Einsatz gelangenden Hybridisierungsbedingungen mit dieser Sonde von hinreichender Kürze ermöglicht wird.

7. Verfahren nach Anspruch 5 oder 6, dadurch gekennzeichnet, daß der Inkubationsschritt bei dieser vorbestimmten Temperatur durchgeführt wird und das Waschen bei jener oder auch einer niedrigeren Temperatur erfolgt.

8. Verfahren nach Anspruch 5 oder 6, dadurch gekennzeichnet, daß der Inkubationsschritt bei einer niedrigeren Temperatur als der vorbestimmten Temperatur durchgeführt wird und daß das Waschen bei der vorbestimmten Temperatur erfolgt.

9. Verfahren nach einem der Ansprüche 5-8, dadurch gekennzeichnet, daß die vorbestimmte Temperatur zwischen 20 und 60°C, insbesondere zwischen 25 und 40°C beträgt und insbesondere 37°C beträgt.

10. Verfahren nach einem der vorstehenden Ansprüche, dadurch gekennzeichnet, daß die Erkennungssonde mindestens 9 Nukleotide enthält.

11. Verfahren nach Anspruch 10, dadurch gekennzeichnet, daß die Erkennungssonde mindestens 15 Nukleotide enthält.

12. Verfahren nach einem der vorstehenden Ansprüche, dadurch gekennzeichnet, daß das aus der Aufnahmesonde und dem gesuchten Target gebildete Hybrid mindestens so stabil ist wie das aus dem Target mit der Erkennungssonde gebildete Hybrid.

13. Verfahren nach einem der vorstehenden Ansprüche, dadurch gekennzeichnet, daß die Erkennungssonde im Hinblick auf die Anzahl der Nukleotide länger ist als die Aufnahmesonde.

14. Verfahren nach einem der vorstehenden Ansprüche, dadurch gekennzeichnet, daß die Aufnahme- und/oder Erkennungssonde α-D-Nukleotide enthält oder aus solchen besteht.

15. Verfahren nach einem der vorstehenden Ansprüche, dadurch gekennzeichnet, daß das Protein Säugeralbumin oder bakterielles Eiweißtoxin ist.

16. Verfahren nach einem der vorstehenden Ansprüche, dadurch gekennzeichnet, daß der Träger in Form von Röhrchen, Kügelchen, Partikeln, oder einer kegelförmigen Pipettierspitze oder in Form von Mikrotiterplatten vorliegt.

17. Verfahren nach einem der vorstehenden Ansprüche, dadurch gekennzeichnet, daß nach der simultanen Sandwich-Hybridisierungsmethode gearbeitet wird.

18. Verfahren nach einem der vorstehenden Ansprüche, dadurch gekennzeichnet, daß das Oligonukleotid entweder eine perfekt homologe Sequenz der Komplementärsequenz des Targets oder eine Sequenz mit einem Gehalt an einer punktuellen Mutation in bezug auf die Komplementärsequenz des Targets darstellt.

19. Verfahren nach einem der vorstehenden Ansprüche, dadurch gekennzeichnet, daß das Target die DNA-Sequenz des Human-Papillomavirus HPV 18 darstellt und die Aufnahmesonde die Sequenz

TCAGAGGAAGAAAACGATGA.

20. Verfahren nach Anspruch 21, dadurch gekennzeichnet, daß die Erkennungssonde die Sequenz TAAGGCAACATTGCAAGACA enthält.

21. Verfahren nach einem der vorstehenden Ansprüche 1-18, dadurch gekennzeichnet, daß das Target eine Messenger-RNA-Sequenz darstellt, die dem ß-Lactamasegen von E. Coli (Tem) entspricht und daß die Aufnahmesonde die folgende Sequenz enthält: 5'-GCACTGCATAATTCTT-3' oder

5'-TACTGTCATGCCATCC-3'

22. Verfahren nach dem vorhergehenden Anspruch, dadurch gekennzeichnet, daß die Erkennungssonde die folgende Sequenz enthält: 5'-GTTGGCCGCAGTGTTAT-3' oder

5'-CACTCATGGTTATGGCA-3'.

23. Verfahren nach einem der Ansprüche 1-18, dadurch gekennzeichnet, daß das Target eine DNA-Sequenz darstellt, die ein ß-Lactamasegen aus E. Coli enthält, und daß die Aufnahmesonde die folgende Sequenz enthält: 5'-GGATGGCATGACAGTA-3' oder

5'-AGAGAATTATGCAGTGC-3'

24. Verfahren nach dem vorhergehenden Anspruch, dadurch gekennzeichnet, daß die Erkennungssonde die folgende Sequenz enthält: 5′-TGCCATAACCATGAGTG-3′ oder

5′-ATAACACTGCGGCCAAC-3′

25. Verfahren nach einem der Ansprüche 1-18, dadurch gekennzeichnet, daß das Target die dem Papillomavirus-Humantyp 6-Gen-E7 entsprechende DNA-Sequenz darstellt und daß die Aufnahmesonde die folgende Sequenz enthält: 5′-CAGTGTACAGAAACA-3′ oder

5′-GAGTGCACAGACGGA-3′

26. Verfahren nach dem vorhergehenden Anspruch, dadurch gekennzeichnet, daß die Erkennungssonde die folgende Sequenz enthält: 5′-GACCCTGTAGGGTTACATT-3′ oder

5′-TGACCTGTTGCTGTGGA-3′

27. Verfahren nach einem der Ansprüche 1-18, dadurch gekennzeichnet, daß das Target die dem Papillomavirus-Humantyp 11-Gen-E7 entsprechende DNA-Sequenz darstellt und daß die Aufnahmesonde die folgende Sequenz enthält: 5′-GAGTGCACAGACGGA-3′ oder

5′-CAACTACAAGACCTTTTGC-3′

28. Verfahren nach dem vorhergehenden Anspruch, dadurch gekennzeichnet, daß die Erkennungssonde die folgende Sequenz enthält: 5′-GACCCTGTAGGGTTACATT-3′ oder

5′-TGACCTGTTGCTGTGGA-3′

29. Verfahren nach einem der Ansprüche 1-18, dadurch gekennzeichnet, daß das Target die den E7-Genen des Papillomavirus-Humantyps 6 oder 11 entsprechende DNA-Sequenz darstellt und daß die Aufnahmesonde die folgende Sequenz aufweist: 5′-AGACAGCTCAGAAGATGAGG-3′ oder

5′-CAGCAACGT(T/C)CGACTGGTTG-3′

30. Verfahren nach dem vorhergehenden Anspruch, dadurch gekennzeichnet, daß die Erkennungssonde die folgende Formel enthält: 5′-GACCCTGTAGGGTTACATT-3′ oder

5′-TGACCTGTTGCTGTGGA-3′

31. Verfahren nach einem der Ansprüche 1-18, dadurch gekennzeichnet, daß das Target die dem Papillomavirus-Humantypl 6-Gen-E7 entsprechende DNA-Sequenz darstellt und daß die Aufnahmesonde die folgende Sequenz aufweist: 5′-TATATGTTAGATTTGCAACC-3′ oder

5′-GACAACTGATCTCTAC-3′

32. Verfahren nach dem vorhergehenden Anspruch, dadurch gekennzeichnet, daß die Erkennungssonde die folgende Formel enthält: 5′-CTCTACGCTTCGGTTGTGC-3′ oder

5′-CCGGACAGAGCCCATTAC-3′

33. Verfahren nach einem der Ansprüche 1-18, dadurch gekennzeichnet, daß das Target die dem Papillomavirus-Humantyp 18-Gen-E7 entsprechende DNA-Sequenz darstellt und daß die Aufnahmesonde die folgende Sequenz aufweist: 5′-TCAGAGGAAGAAAACGATGA-3′ oder

5′-ATGTCACGAGCAATTAAGCG-3′

34. Verfahren nach dem vorhergehenden Anspruch, dadurch gekennzeichnet, daß die Erkennungssonde die folgende Sequenz aufweist: 5'-GTATTGCATTTAGAGCCCCA-3' oder

5′-TAAGGCAACATTGCAAGACA-3′

35. Verfahren nach einem der Ansprüche 1-18, dadurch gekennzeichnet, daß das Target die dem Papillomavirus-Humantypl 8 entsprechende DNA-Sequenz darstellt und daß die Aufnahmesonde die folgende Sequenz aufweist:

5′-TCAGAGGAAGAAAACGATGA-3′

36. Verfahren nach dem vorstehenden Anspruch, dadurch gekennzeichnet, daß die Erkennungssonde die folgende Sequenz aufweist:

5′-TAAGGCAACATTGCAAGACA-3′

37. Verfahren nach einem der Ansprüche 1-18, dadurch gekennzeichnet, daß das Target die dem größeren externen Membranproteingen (MOMP) von Chlamydia trachomatis entsprechende DNA-Sequenz darstellt und daß die Aufnahmesonde die folgende Sequenz enthält: 5′-GCCGCTTTGAGTTCTGCTTCC-3′ oder

5′-CTTGCAAGCTCTGCCTGTGG-3′

38. Verfahren nach dem vorhergehenden Anspruch, dadurch gekennzeichnet, daß die Erkennungssonde die folgende Sequenz enthält: 5′-AATCCTGGCTGAACCAAGCCT-3′ oder

5′-AAGGTTTCGGCGGAGATCCT-3′

39. Verfahren nach einem der Ansprüche 1-18, dadurch gekennzeichnet, daß das Target eine Sequenz darstellt, die in Form der DNA der gag-Region des Retrovirus' HIV1 entspricht und daß die Aufnahmesonde die folgende Formel aufweist:

5′-AATGTTAAAAGAGAC-3′

40. Verfahren nach dem vorhergehenden Anspruch, dadurch gekennzeichnet, daß die Erkennungssonde die folgende Sequenz enthält:

5′-GAAGCTGCAGAATGGGA-3′

41. Verfahren nach einem der Ansprüche 1-18, dadurch gekennzeichnet, daß das Target eine Sequenz darstellt, die in Form der DNA der gag-Region des Retrovirus' HIV2 entspricht und daß die Aufnahmesonde die folgende Sequenz aufweist:

5′-CTTACCAGCGGGGCAGC-3′

42. Verfahren nach dem vorhergehenden Anspruch, dadurch gekennzeichnet, daß die Erkennungssonde die folgende Sequenz enthält:

5′-GAAGCTGCAGAATGGGA-3′

43. Verfahren nach einem der Ansprüche 1-18, dadurch gekennzeichnet, daß das Target eine Sequenz darstellt, die in Form der DNA der env-Region des Retrovirus' HIV1 entspricht und daß die Aufnahmesonde die folgende Sequenz aufweist:

5′-TGTCTGGTATAGTGCA-3′

44. Verfahren nach dem vorhergehenden Anspruch, dadurch gekennzeichnet, daß die Erkennungssonde die folgende Sequenz enthält:

5′-AACAATTTGCTGAGGGCTAT-3′

45. Verfahren nach einem der Ansprüche 1-18, dadurch gekennzeichnet, daß das Target eine Sequenz darstellt, die in Form der DNA der env-Region des Retrovirus' HIV2 entspricht und daß die Aufnahmesonde die folgende Sequenz enthält:

5′-GCAACAGCAACAGCTGTTGGA-3′

46. Verfahren nach dem vorhergehenden Anspruch, dadurch gekennzeichnet, daß die Erkennungssonde die folgende Sequenz enthält:

5′-GGTCAAGAGACAACAAGAA-3′

47. Verfahren nach einem der Ansprüche 1-18, dadurch gekennzeichnet, daß das Target eine Sequenz darstellt, die in Form der DNA der pol-Region des Retrovirus' HIV1 entspricht und daß die Aufnahmesonde die folgende Sequenz enthält:

5′-GACAATGGCAGCAATTTCACC-3′

48. Verfahren nach dem vorhergehenden Anspruch, dadurch gekennzeichnet, daß die Erkennungssonde die folgende Sequenz enthält:

5′-GCCTGTTGGTGGGC-3′

49. Verfahren nach einem der Ansprüche 1-18, dadurch gekennzeichnet, daß das Target die DNA-Sequenz darstellt, die dem Gen der ß-Glucuronidase-Region von E. Coli entspricht und daß die Aufnahmesonde die folgende Sequenz aufweist: 5'-TGGCTTCTGTCAACGCTGTT-3' oder

5′-ATGCGATCTATATCACGCTG-3′

50. Verfahren nach dem vorhergehenden Anspruch, dadurch gekennzeichnet, daß die Erkennungssonde die folgende Sequenz enthält: 5′-GATCGCGGTGTCAGTTCTTT-3′ oder

5'-TTCCATGGGTTTCTCACAGA-3'

51. Verfahren nach einem der Ansprüche 1-18, dadurch gekennzeichnet, daß das Target die DNA-Sequenz darstellt, die dem β-Humanglobingen entspricht und daß die Aufnahmesonde die folgende Sequenz aufweist: 5′-TGTTTACGCAGTCTGCCTAG-3′ oder

5′-CACATATTGACCAAATCAGG-3′

52. Verfahren nach dem vorhergehenden Anspruch, dadurch gekennzeichnet, daß die Erkennungssonde die folgende Sequenz aufweist: 5′-GTATCATGCCTCTTTGCACC-3′ oder

5′-TTTCTGGGTTAAGGCAATAGC-3′

53. Verfahren nach einem der Ansprüche 1-18, dadurch gekennzeichnet, daß das Target die DNA-Sequenz darstellt, die dem ras-Onkogen entspricht und daß die Aufnahmesonde die folgende Sequenz enthält: 5'-CAGTGCCATGAGAGACCAAT-3' oder

5′-CGACGCAGCCATGGTCGATGC-3′

54. Verfahren nach dem vorhergehenden Anspruch, dadurch gekennzeichnet, daß die Erkennungssonde die folgende Sequenz enthält: 5'-TTCCTCTGTGTATTTGCCAT-3' oder

5′-ACATGAGGACAGGCGAAGGC-3′

55. Verfahren nach einem der Ansprüche 1-18, dadurch gekennzeichnet, daß das Target die DNA-Sequenz vom HPV-Typ-18 darstellt und daß die Aufnahmesonde die folgende Sequenz aufweist:

5′-TCAGAGGAAGAAAACGATGA-3′

56. Verfahren nach dem vorhergehenden Anspruch, dadurch gekennzeichnet, daß die Erkennungssonde die folgende (a-Nukleotid)-Sequenz enthält:

α-Oligonukleotid 5′-ACCCCGAGATT

57. Verfahren nach einem der Ansprüche 1-18, dadurch gekennzeichnet, daß das Target eine Sequenz darstellt, die der NEF-Region des Retrovirus' HIV1 entspricht und daß die Aufnahmesonde die folgende Sequenz aufweist:

5′-GAGGAGGTIGGTTTTCCAGTCA-3′

58. Verfahren nach dem vorhergehenden Anspruch, dadurch gekennzeichnet, daß die Erkennungssonde die Sequenz

5′-GGATGGCCTICTITAAGGGAAAGAATG-3′

59. Verfahren nach einem der Ansprüche 1-18, dadurch gekennzeichnet, daß das Target eine Sequenz darstellt, die der VPR-Region des Retrovirus' HIV1 entspricht und daß die Aufnahmesonde die Sequenz 5′-TATGAAACTTATGGGGATAC-3′ enthält.

60. Verfahren nach dem vorhergehenden Anspruch, dadurch gekennzeichnet, daß die Erkennungssonde die Sequenz 5′-GAAGCTGTTAGACATTTTCCTAG-3′ enthält.

61. Verfahren nach einem der Ansprüche 1-18, dadurch gekennzeichnet, daß das Target eine Sequenz darstellt, die der pol-Region des Retrovirus' HIV1 entspricht und daß die Aufnahmesonde die Sequenz 5′-TGAACAAGTAGATAAATTAG-3′ enthält.

62. Verfahren nach dem vorhergehenden Anspruch, dadurch gekennzeichnet, daß die Erkennungssonde die Sequenz 5′-GGAATCAGIAAAGTACTATT-3′ enthält.

## Claims

1. Method for detecting a single-stranded target nucleotide sequence in a sample containing it or capable of containing it, by the sandwich hybridization technique, characterized in that it comprises the steps consisting:
(a) in incubating the sample:
(i) with a capture probe attached to a solid support, the said solid support being produced from a hydrophobic material and the said capture probe being composed of an oligonucleotide capable of attaching by adsorption onto the said solid support or via an oligonucleotide bound to a protein by covalent bonding, the said oligonucleotide being attached by adsorption onto the said solid support either directly or via the said protein when it is present, the said oligonucleotide having from 9 to 30 nucleotides, it being understood that when the said capture probe is composed of an oligonucleotide not bound to a protein, the oligonucleotide comprises at least 11 oligonucleotides, and
(ii) with a detection probe labelled with a nonradioactive marker, the capture and detection probes being capable of hybridizing respectively with two nonoverlapping regions of the desired nucleotide-target sequence, then
(b) in washing the solid support in order to remove the reagents not attached by hybridization, and
(c) in detecting the target sequence by means of a reaction for revealing the marker attached to the support.

2. Method according to Claim 1, characterized in that the said hydrophobic material is a hydrophobic polymer.

3. Method according to Claim 2, characterized in that the said polymer is a styrene polymer or copolymer.

4. Method according to Claim 1, characterized in that the said oligonucleotide contains at most, and in particular at most 20 nucleotides.

5. Method according to any one of the preceding claims, characterized in that the procedure is carried out at a predetermined temperature, and in that capture and detection probes are chosen which are of a sufficiently long size and of a sequence such that the hybrid which each probe forms with the desired target is stable at the said temperature, under the hybridization conditions used.

6. Method according to Claim 5, characterized in that the capture probe and/or the detection probe is sufficiently short and of a sequence such that only the presence of a perfectly homologous complementary sequence in the target makes it possible to form with the said sufficiently short probe a hybrid stable at the said temperature under the hybridization conditions used.

7. Method according to Claim 5 or 6, characterized in that the incubation stage is performed at the said predetermined temperature and in that the washing is performed at the said temperature or at a lower temperature.

8. Method according to Claim 5 or 6, characterized in that the incubation stage is performed at a temperature below the said predetermined temperature, and in that the washing is performed at the said predetermined temperature.

9. Method according to any one of Claims 5 to 8, characterized in that the said predetermined temperature is between 20 and 60°C, especially between 25 and 40°C, and is in particular equal to 37°C.

10. Method according to any one of the preceding claims, characterized in that the detection probe contains at least 9 nucleotides.

11. Method according to Claim 10, characterized in that the detection probe contains at least 15 nucleotides.

12. Method according to any one of the preceding claims, characterized in that the hybrid formed by the capture probe and the desired target is less stable than the hybrid formed by the said target with the detection probe.

13. Method according to any one of the preceding claims, characterized in that the detection probe is longer, in terms of number of nucleotides, than the capture probe.

14. Method according to any one of the preceding claims, characterized in that the capture probe and/or the detection probe contains or is composed of alpha-D-nucleotides.

15. Method according to any one of the preceding claims, characterized in that the said protein is a mammalian albumin or a bacterial protein toxin.

16. Method according to any one of the preceding claims, characterized in that the support is in the form of a tube, bead, particles or pipette cone, or in the form of a microtitre plate.

17. Method according to any one of the preceding claims, characterized in that the procedure is carried out according to the simultaneous sandwich hybridization technique.

18. Method according to any one of the preceding claims, characterized in that the said oligonucleotide is either a sequence which is perfectly homologous to the sequence complementary to the target, or a sequence containing a point mutation compared with the sequence complementary to the target.

19. Method according to any one of the preceding claims, characterized in that the target is the human papillomavirus HPV 18 DNA sequence and in that the capture probe contains the sequence

TCAGAGGAAGAAAACGATGA

20. Method according to Claim 21, characterized in that the detection probe contains the sequence:

TAAGGCAACATTGCAAGACA

21. Method according to any one of Claims 1 to 18, characterized in that the target is a messenger RNA sequence corresponding to the E. coli (TEM) beta-lactamase gene and in that the capture probe contains the following sequence: 5'-GCACTGCATAATTCTT-3' or

5′-TACTGTCATGCCATCC-3′

22. Method according to the preceding claim, characterized in that the detection probe contains the following sequence: 5'-GTTGGCCGCAGTGTTAT-3' or

5'-CACTCATGGTTATGGCA-3'

23. Method according to any one of Claims 1 to 18, characterized in that the target is a DNA sequence containing the E. coli beta-lactamase gene and in that the capture probe contains the following sequence: 5′-GGATGGCATGACAGTA-3′ or

5'-AGAGAATTATGCAGTGC-3'

24. Method according to the preceding claim, characterized in that the detection probe contains the following sequence: 5'-TGCCATAACCATGAGTG-3' or

5'-ATAACACTGCGGCCAAC-3'

25. Method according to any one of Claims 1 to 18, characterized in that the target is the DNA sequence corresponding to the type 6 human papillomavirus E7 gene and in that the capture probe contains the following sequence: 5'-CAGTGTACAGAAACA-3' or

5′-GAGTGCACAGACGGA-3′

26. Method according to the preceding claim, characterized in that the detection probe contains the following sequence: 5'-GACCCTGTAGGGTTACATT-3' or

5'-TGACCTGTTGCTGTGGA-3'

27. Method according to any one of Claims 1 to 18, characterized in that the target is the DNA sequence corresponding to the type 11 human papillomavirus E7 gene and in that the capture probe contains the following sequence: 5'-GAGTGCACAGACGGA-3' or

5'-CAACTACAAGACCTTTTGC-3'

28. Method according to the preceding claim, characterized in that the detection probe contains the following sequence: 5'-GACCCTGTAGGGTTACATT-3' or

5′-TGACCTGTTGCTGTGGA-3′

29. Method according to any one of Claims 1 to 18, characterized in that the target is the DNA sequence corresponding to the type 6 or 11 human papillomavirus E7 genes, and in that the capture probe contains the following sequence: 5'-AGACAGCTCAGAAGATGAGG-3' or

5'-CAGCAACGT(T/C)CGACTGGTTG-3'

30. Method according to the preceding claim, characterized in that the detection probe contains the following sequence: 5'-GACCCTGTAGGGTTACATT-3' or

5'-TGACCTGTTGCTGTGGA-3'

31. Method according to any one of Claims 1 to 18, characterized in that the target is the DNA sequence corresponding to the type 16 human papillomavirus E7 gene and in that the capture probe contains the following sequence: 5'-TATATGTTAGATTTGCAACC-3' or

5'-GACAACTGATCTCTAC-3'

32. Method according to the preceding claim, characterized in that the detection probe contains the following sequence: 5'-CTCTACGCTTCGGTTGTGC-3' or

5'-CCGGACAGAGCCCATTAC-3'

33. Method according to any one of the Claims 1 to 18, characterized in that the target is the DNA sequence corresponding to the type 18 human papillomavirus E7 gene and in that the capture probe contains the following sequence: 5'-TCAGAGGAAGAAAACGATGA-3' or

5'-ATGTCACGAGCAATTAAGCG-3'

34. Method according to the preceding claim, characterized in that the detection probe contains the following sequence: 5'-GTATTGCATTTAGAGCCCCA-3' or

5′-TAAGGCAACATTGCAAGACA-3′

35. Method according to any one of Claims 1 to 18, characterized in that the target is the DNA sequence corresponding to the type 18 human papillomavirus and in that the capture probe contains the following sequence:

5'-TCAGAGGAAGAAAACGATGA-3'

36. Method according to the preceding claim, characterized in that the detection probe contains the following sequence:

5'-TAAGGCAACATTGCAAGACA-3'

37. Method according to any one of Claims 1 to 18, characterized in that the target is the DNA sequence corresponding to the Chlamydia trachomatis major outer membrane protein (MOMP) gene and in that the capture probe contains the following sequence: 5-GCCGCTTTGAGTTCTGCTTCC-3' or

5′-CTTGCAAGCTCTGCCTGTGG-3′

38. Method according to the preceding claim, characterized in that the detection probe contains the following sequence: 5'-AATCCTGGCTGAACCAAGCCT-3' or

5'-AAGGTTTCGGCGGAGATCCT-3'

39. Method according to any one of Claims 1 to 18, characterized in that the target is a sequence corresponding, in DNA form, to the HIV1 retrovirus gag region and in that the capture probe contains the following sequence:

5′-AATGTTAAAAGAGAC-3′

40. Method according to the preceding claim, characterized in that the detection probe contains the following sequence:

5-GAAGCTGCAGAATGGGA-3'

41. Method according to any one of Claims 1 to 18, characterized in that the target is a sequence corresponding, in DNA form, to the HIV2 retrovirus gag region and in that the capture probe contains the following sequence:

5'-CTTACCAGCGGGGCAGC-3'

42. Method according to the preceding claim, characterized in that the detection probe contains the following sequence:

5'-GAAGCTGCAGAATGGGA-3'

43. Method according to any one of Claims 1 to 18, characterized in that the target is a sequence corresponding, in DNA form, to the HIV1 retrovirus env. region and in that the capture probe contains the following sequence:

5'-TGTCTGGTATAGTGCA-3'

44. Method according to the preceding claim, characterized in that the detection probe contains the following sequence:

5'-AACAATTTGCTGAGGGCTAT-3'

45. Method according to any one of Claims 1 to 18, characterized in that the target is a sequence corresponding, in DNA form, to the HIV2 retrovirus env. region and in that the capture probe contains the following sequence:

5'-GCAACAGCAACAGCTGTTGGA-3'

46. Method according to the preceding claim, characterized in that the detection probe contains the following sequence:

5'-GGTCAAGAGACAACAAGAA-3'

47. Method according to any one of Claims 1 to 18, characterized in that the target is a sequence corresponding, in DNA form, to the HIV1 retrovirus pol region and in that the capture probe contains the following sequence:

5'-GACAATGGCAGCAATTTCACC-3'

48. Method according to the preceding claim, characterized in that the detection probe contains the following sequence:

5'-GCCTGTTGGTGGGC-3'

49. Method according to any one of Claims 1 to 18, characterized in that the target is the DNA sequence corresponding to the gene for the E. coli betaglucoronidase region and in that the capture probe contains the following sequence: 5'-TGGCTTCTGTCAACGCTGTT-3' or

5′-ATGCGATCTATATCACGCTG-3′

50. Method according to the preceding claim, characterized in that the detection probe contains the following sequence: 5'-GATCGCGGTGTCAGTTCTTT-3' or

5'-TTCCATGGGTTTCTCACAGA-3'

51. Method according to any one of Claims 1 to 18, characterized in that the target is the DNA sequence corresponding to the gene for the human beta-globin region and in that the capture probe contains the following sequence: 5'-TGTTTACGCAGTCTGCCTAG-3' or

5'-CACATATTGACCAAATCAGG-3'

52. Method according to the preceding claim, characterized in that the detection probe contains the following sequence: 5'-GTATCATGCCTCTTTGCACC-3' or

5'-TTTCTGGGTTAAGGCAATAGC-3'

53. Method according to any one of Claims 1 to 18, characterized in that the target is the DNA sequence corresponding to the ras oncogene and in that the capture probe contains the following sequence: 5'-CAGTGCCATGAGAGACCAAT-3' or

5'-CGACGCAGCCATGGTCGATGC-3'

54. Method according to the preceding claim, characterized in that the detection probe contains the following sequence: 5'-TTCCTCTGTGTATTTGCCAT-3' or

5'-ACATGAGGACAGGCGAAGGC-3'

55. Method according to any one of Claims 1 to 18, characterized in that the target is the type 18 HPV DNA sequence and in that the capture probe contains the following sequence:

5'-TCAGAGGAAGAAAACGATGA-3'

56. Method according to the preceding claim, characterized in that the detection probe contains the following (alpha-nucleotide) sequence:

alpha-oligonucleotide 5'-ACCCCGAGATT

57. Method according to any one of Claims 1 to 18, characterized in that the target is a sequence corresponding to the HIV1 retrovirus NEF region, and in that the capture probe contains the sequence:

5'-GAGGAGGTIGGTTTTCCAGTCA-3'

58. Method according to the preceding claim, characterized in that the detection probe contains the sequence:

5'-GGATGGCCTICTITAAGGGAAAGAATG-3'

59. Method according to any one of Claims 1 to 18, characterized in that the target is a sequence corresponding to the HIV1 retrovirus VPR region, and in that the capture probe contains the sequence:

5'-TATGAAACTTATGGGATAC-3'

60. Method according to the preceding claim, characterized in that the detection probe contains the sequence:

5'-GAAGCTGTTAGACATTTTCCTAG-3'

61. Method according to any one of Claims 1 to 18, characterized in that the target is a sequence corresponding to the HIV1 retrovirus POL region, and in that the capture probe contains the sequence:

5'-TGAACAAGTAGATAAATTAG-3'

62. Method according to the preceding claim, characterized in that the detection probe contains the sequence:
